# EUROPEAN PATENT SPECIFICATION

(11) **EP 2 195 021 B1**
(45) Date of publication and mention of the grant of the patent: **29.02.2012**
(21) Application number: 08803235.4
(22) Date of filing: 26.08.2008
(51) Int. Cl.: A61K 39/245, C12N 7/04, C12N 15/38, C12N 15/85, C12N 15/86, A61K 39/205

(54) **A RECOMBINANT KOI HERPESVIRUS (KHV) OR CYPRINID HERPESVIRUS 3 (CYHV-3) AND A VACCINE FOR THE PREVENTION OF A DISEASE CAUSED BY KHV/CYHV-3 IN CYPRINUS CARPIO CARPIO OR CYPRINUS CARPIO KOI**
REKOMBINANTER KOI-HERPESVIRUS (KHV) ODER CYPRINID-HERPESVIRUS 3 (CYHV-3) UND IMPFSTOFF ZUR PRÄVENTION EINER VON KHV/CYHV-3 BEI CYPRINUS CARPIO CARPIO ODER CYPRINUS CARPIO KOI VERURSACHTEN ERKRANKUNG
HERPÈSVIRUS KOÏ (KHV) OU HERPÈSVIRUS 3 DE CYPRINIDÉ (CYHV-3) RECOMBINÉ ET VACCIN POUR PRÉVENIR UNE MALADIE PROVOQUÉE PAR KHV/CYHV-3 CHEZ LA CARPE JAPONAISE OU CARPE KOÏ

(30) Priority: 28.08.2007 EP 07115093; 16.11.2007 EP 07120939
(43) Date of publication of application: 16.06.2010
(73) Proprietor: Université de Liège, 4031 Angleur (BE)
(72) Inventor: COSTES, Bérénice, B-4000 Liège (BE); LIEFFRIG, François, B-6900 Marloie (BE); VANDERPLASSCHEN, Alain, B-4000 Liège (BE)
(74) Representative: Keus, Jacobus Albertus Ronald
(86) International application number: PCT/EP2008/061168
(87) International publication number: WO 2009/027412

(56) References cited:
- WO-A-2004/061093
- PERELBERG A ET AL: "Protection of cultured Cyprinus carpio against a lethal viral disease by an attenuated virus vaccine" VACCINE, BUTTERWORTH SCIENTIFIC. GUILDFORD, GB, vol. 23, no. 26, 16 May 2005 (2005-05-16), pages 3396-3403, XP004852130 ISSN: 0264-410X
- AOKI TAKASHI ET AL: "Genome sequences of three koi herpesvirus isolates representing the expanding distribution of an emerging disease threatening koi and common carp worldwide" JOURNAL OF VIROLOGY, vol. 81, no. 10, May 2007 (2007-05), pages 5058-5065, XP002465776 ISSN: 0022-538X cited in the application
- BERCOVIER HERVE ET AL: "Cloning of the koi herpesvirus (KHV) gene encoding thymidine kinase and its use for a highly sensitive PCR based diagnosis" BMC MICROBIOLOGY, BIOMED CENTRAL, LONDON, GB, vol. 5, no. 1, 17 March 2005 (2005-03-17), page 13, XP021002610 ISSN: 1471-2180
- YASUMOTO SHINYA ET AL: "Oral immunization of common carp with a liposome vaccine fusing koi herpesvirus antigen" FISH PATHOLOGY, vol. 41, no. 4, December 2006 (2006-12), pages 141-145, XP002465774 ISSN: 0388-788X
- KANELLOS ET AL: "DNA vaccination can protect Cyprinus Carpio against spring viraemia of carp virus" VACCINE, BUTTERWORTH SCIENTIFIC. GUILDFORD, GB, vol. 24, no. 23, 5 June 2006 (2006-06-05), pages 4927-4933, XP005464750 ISSN: 0264-410X
- WAGNER MARKUS ET AL: "Herpesvirus genetics has come of age" TRENDS IN MICROBIOLOGY, vol. 10, no. 7, July 2002 (2002-07), pages 318-324, XP002465775 ISSN: 0966-842X cited in the application
- COSTES B ET AL: "Cloning of the koi herpesvirus genome as an infectious bacterial artificial chromosome demonstrates that disruption of the thymidine kinase locus induces partial attenuation in Cyprinus carpio koi" JOURNAL OF VIROLOGY, vol. 82, no. 10, May 2008 (2008-05), pages 4955-4964, XP002505812 ISSN: 0022-538X

### SEQUENCE LISTING

<110> Université de Liège
<120> A RECOMBINANT KOI HERPESVIRUS (KHV) OR CYPRINID HERPESVIRUS 3 (CYHV-3) AND A VACCINE FOR THE PREVENTION OF A DISEASE CAUSED BY KHV/CYHV-3 IN CYPRINUS CARPIO CARPIO OR CYPRINUS CARPIO KOI
<130> P22025WO
<150> EP 07 115 093.2 <151> 2007-08-28
<150> EP 07 120 939.9 <151> 2007-11-16
<160> 10
<170> PatentIn version 3.3
<210> 1
   <211> 21
   <212> DNA
   <213> Artificial
<220>
   <223> Primer
<400> 1
   atggctatgc tggaactggt g 21
<210> 2
   <211> 20
   <212> DNA
   <213> Artificial
<220>
   <223> Primer
<400> 2
   ctcaacaggg aagagtggcg 20
<210> 3
   <211> 21
   <212> DNA
   <213> Artificial
<220>
   <223> Primer
<400> 3
   atggctatgc tggaactggt g 21
<210> 4
   <211> 20
   <212> DNA
   <213> Artificial
<220>
   <223> Primer
<400> 4
   ctcaacaggg aagagtggcg 20
<210> 5
   <211> 74
   <212> DNA
   <213> Artificial
<220>
   <223> Primer
<400> 5
   atgaagctgc ttgtgattct gggtcttgga ctctgctacc tggctgttca cctgttgaca 60
   attaatcatc ggca 74
<210> 6
   <211> 70
   <212> DNA
   <213> Artificial
<220>
   <223> Primer
<400> 6
   tcaacttctc tttggttttc tgcacatatt cgtccccccc acggttttca tcagcactgt 60
   cctgctcctt 70
<210> 7
   <211> 74
   <212> DNA
   <213> Artificial
<220>
   <223> Primer
<400> 7
   atgaaacctc tgggtctttt tgtttctgtg ctcgggctgc ttgccctgtc cctgttgaca 60
   attaatcatc ggca 74
<210> 8
   <211> 70
   <212> DNA
   <213> Artificial
<220>
   <223> Primer
<400> 8
   tcataggacg ccatcggttg agttcgctgc ggctgcgact cccagtcctc tcagcactgt 60
   cctgctcctt 70
<210> 9
   <211> 69
   <212> DNA
   <213> Artificial
<220>
   <223> Primer
<400> 9
   atggaacccg agcctcgaca gcagaaccgc gagctccctt tcatgcctgt tgacaattaa 60
   tcatcggca 69
<210> 10
   <211> 69
   <212> DNA
   <213> Artificial
<220>
   <223> Primer
<400> 10
   tcaagaccat aaagtgggaa ggtgtcgtcg aagccgtctc tggagtcgtt cagcactgtc 60
   ctgctcctt 69

## Description

### Field of the invention

The present invention refers to a recombinant Koi herpesvirus (KHV) and a vaccine for the prevention of a disease caused by Koi herpesvirus/Cyprinid herpesvirus 3 in *Cyprinus carpio carpio* or *Cyprinus carpio koi.*

### Background of the invention

Common carp *(Cyprinus carpio carpio)* is the most widely cultivated fish for human consumption mainly in Asia, Europe, and the Middle East. In contrast, the Koi *(Cyprinus carpio koi)* subspecies is cultivated as an expensive beautiful and colorful pet fish for personal pleasure or competitive showing especially in Japan but also worldwide. A virus causing a lethal disease in common and Koi carps, initially called Koi herpesvirus disease (KHVD), was detected in 1996 in the United Kingdom. The virus was then rapidly identified as the cause of mass mortality among Koi and common carps in Israel, the USA, and Germany. Intensive culture of common carps, Koi shows and international trading have unfortunately contributed to the rapid global spread of this highly contagious and extremely virulent disease. Since its emergence, KHVD has caused severe financial and economic losses in both Koi and carp culture industries worldwide.

Initial characterization of the virus showed a herpes-like structure with an envelope and an icosahedral electron-dense core of 100-110 nm surrounded by a tegument-like structure. The genome of the virus comprises linear double-stranded DNA (dsDNA) of ∼295 kb similar to that of Cyprinid herpesvirus 1 (CyHV-1) but larger than those of other *Herpesviridae* members generally ranging from 125 to 240 kb in size. The sequence of KHV genome has been published very recently (Aoki et al., J Virol, 81, pages 5058-5065 (2007)). The KHV genome contains a significant number of DNA sequences without homology to any other known viral sequences. Moreover, it contains highly divergent DNA sequences encoding polypeptides, which resemble those of several dsDNA viruses, like herpesvirus, poxvirus, iridovirus and other large DNA viruses.

The unique characteristics of this virus have led to three different nomenclatures: firstly, Koi herpesvirus (KHV) according to its morphological manifestation; secondly, carp interstitial nephritis and gill necrosis virus (CNGV) according to its pathogenic effects in fish; and lastly Cyprinid herpesvirus 3 (CyHV-3) according to gene content similarity with CyHV-1 and CyHV-2. The latter nomenclature has been further supported by the recent sequencing of the full length of the viral genome. However, hereinafter the name KHV will be used.

KHV bears a genome of approximately 295 kb which represents the largest genome ever identified among *Herpesviridae* members. Since the first isolation of KHV in 1996, no information is available about the role of individual genes in KHV pathogenesis and in the biology of the infection of the natural host.

An attenuated vaccine candidate has been described in the international patent application WO 2004/061093 A1. However, this vaccine candidate exhibits two major defaults. Firstly, the attenuation is the consequence of random mutations that occurred during viral replication *in vitro.* Consequently, the determinism of the attenuation is unknown and reversion to a fully pathogenic phenotype can not be excluded. Secondly, the vaccine strain exhibits residual virulence in small juvenile fishes killing up to 20% of vaccinated subjects.

### Problem and solution

Since its first isolation in 1996, an increasing number of studies have been devoted to KHV. They reported data related to viral gene content, to viral pathogenesis, to the epidemiology of the infection, to the diagnostic of the infection and to control methods. However, to date no KHV recombinant strain has been produced, and consequently, the role of individual genes in KHV pathogenesis and in the biology of infection of the natural host is unknown. Similarly, the absence of KHV recombinant strains can explain the lack of safe and efficacious attenuated recombinant vaccines for the control of the disease on the market.

Applied and fundamental researches on KHV require production of recombinant virus. Recently, manipulation of large herpesvirus genomes has been facilitated by the use of bacterial artificial chromosomes (BAC) vectors (Messerle et al., Proc Natl Acad Sci U S A, 94, 14759-14763 (1997); Wagner et al., Trends Microbiol, 10, 318-324 (2002)). These vectors allow the maintenance and efficient mutagenesis of the viral genome in *Escherichia coli* (*E.coli*) followed by the reconstitution of progeny virions by transfection of the BAC plasmid into permissive eukaryotic cells. To date, the genomes of several herpesviruses have been successfully propagated as infectious BAC clones, including Human Cytomegalovirus (HCMV) which represents the largest herpesvirus genome cloned as a BAC to date (230 Kb) (Borst et al., J Virol, 73, 8320-8329 (1999)).

The object of the present invention therefore was to provide a strain or construct of KHV as vaccine which can be used for the development of safe and efficacious attenuated vaccines to control KHV infection. A further object was to provide a DNA sequence and a vector derived from KHV for the transfer of polynucleotide sequences, i.e. genetic material, into whole fish or fish cells.

The object was solved by a recombinant Koi herpesvirus (KHV), which is immunogenic in fish, preferably in carps, more preferably in *Cyprinus carpio,* even further preferred in *Cyprinus carpio carpio* and/or *Cyprinus carpio koi,* as defined by the claims.

Surprisingly, the recombinant Koi herpesvirus of the present invention, which is deficient in at least the thymidine kinase gene or ORF140: putative thymidylate kinase gene which contributes to virulence, shows a strongly reduced or even no mortality of carps when infected with this herpesvirus and provides immunity against wild-type Koi herpesvirus.

So far, different groups of scientists have attempted for several years to clone the KHV genome as a BAC and failed. They only obtained clones with a reduced genome. These problems may be due to the size of the KHV genome, which is larger than that of any other herpesvirus cloned so far: the largest herpesvirus cloned in the state of art has a size of 235 kb, while the KHV herpesvirus has a size in the range of 290 to 300 kb. Further problems for cloning of this virus were due to the many repetitive sequences comprised in the genome, which may result in unstable clones and reduced size. Therefore, the present invention provides for the first time a full-length infectious BAC clone of the KHV genome, having a size in the range of 290 to 300 kb. The present invention is also the first to produce attenuated recombinants for an herpesvirus genome having a size in the range of 290 to 300 kb. In a further embodiment, the BAC vector sequence may be excised from the herpesvirus genome of the recombinant Koi herpesvirus thereby leaving a heterologous sequence at the excision site or former insertion site in the herpesvirus genome. This BAC vector sequence excised construct of the recombinant Koi herpesvirus is also deficient in at least one gene which contributes to virulence and shows the desired properties, as outlined below.

The recombinant Koi herpesvirus according to the present invention in either form, the KHV BAC clone and the above mentioned KHV construct where the BAC vector sequence is excised from the herpesvirus genome may be used for further manipulation involving for example genetic engineering techniques in order to make the genome deficient in specific genes. By that the size of the genome may strongly be decreased, in case of plurality of viral gene deletion. A derivative obtained therefore will still be within the scope of the present invention.

The recombinant Koi herpesvirus according to the present invention has the capability to confer immunity on fish, preferably on carps, more preferably on *Cyprinus carpio,* even further preferred on *Cyprinus carpio carpio* and/or *Cyprinus carpio koi,* against a disease caused by Koi herpesvirus (KHV).

The recombinant Koi herpesvirus according to the present invention is deficient in at least one gene which contributes to virulence selected from the group consisting of thymidine kinase gene; ORF140: putative thymidylate kinase gene, or any combination thereof. The recombinant Koi herpesvirus according to the present invention may be deficient in viral thymidine kinase gene and at least one further gene which contributes to virulence selected from the group consisting of ORF12: putative tumor necrosis factor (TNF) receptor gene; ORF 16: putative G-protein coupled receptor (GPCR) gene; ORF 134: putative interleukin 10 homologue gene; ORF140: putative thymidylate kinase gene, or any combination thereof. The recombinant Koi herpesvirus may be deficient in all of above mentioned genes. Moreover, the recombinant Koi herpesvirus may be deficient in further viral genes.

More preferred, the recombinant Koi herpesvirus (KHV) is deficient in thymidine kinase gene and ORF140: putative thymidylate kinase gene.

In a further preferred embodiment of the present invention the recombinant Koi herpesvirus is in a live form. Preferably, the recombinant Koi herpesvirus has the capability to reconstitute infectious particles when introduced into permissive eukaryotic cells or fish individuals, preferably in carps, more preferably in *Cyprinus carpio,* even further preferred in *Cyprinus carpio carpio* and/or *Cyprinus carpio koi.*

Further preferred, the recombinant Koi herpesvirus is in an attenuated form.

The recombinant Koi herpesvirus according to the present invention induces a mortality rate of 40 % or less, preferably of 20 % or less, more preferred of 10 % or less, further preferred of 1% or less, further preferred 0.1% or less and most preferred will induce a mortality rate of 0.0% in fish, preferably in carps, more preferred *Cyprinus carpio carpio* or *Cyprinus carpio Koi* infected with said herpesvirus.

In an alternative embodiment of the present invention the recombinant Koi herpesvirus is in inactivated and non-replicative form or in deficient attenuated form. A non-replicative recombinant strain could be produced by deletion of a KHV gene essential for replication. Such a deleted virus will be cultured on a permissive cell line stably expressing the deleted gene (transcomplementation). This approach has been used successfully for different herpesviruses such as gE deleted Suid herpesvirus 1 (Aujeszky virus) and Bovine herpesvirus 1. Any gene which contributes to replication may be made deficient in order to receive a non-replicating recombinant Koi herpesvirus. With other words, any gene which inactivation is responsible to receive a non-replicative recombinant Koi herpesvirus can be deleted. Preferably, the genes of the recombinant KHV according to the present invention that could be deleted in order to provide a non-replicative form are selected from the group consisting of:
ORF 25, ORF 31, ORF 32, ORF 34, ORF 35, ORF 42, ORF 43, ORF 45, ORF 51, ORF 57, ORF 59, ORF 60, ORF 62, ORF 65, ORF 66, ORF 68, ORF 70, ORF 72, ORF 78, ORF 81, ORF 84, ORF 89, ORF 90, ORF 92, ORF 95, ORF 97, ORF 99, ORF 108, ORF 115, ORF 131, ORF 132, ORF 136, ORF 137, ORF 148, ORF 149.

"Deficient attenuated form" means that the recombinant Koi herpesvirus has the capability to infect cells or fish individuals (e.g. *Cyprinus carpio, Cyprinus carpio carpio* or *Cyprinus carpio koi)* but is not able to replicate.

Further preferred, the recombinant Koi herpesvirus according to the present invention comprises a BAC (bacterial artificial chromosome) vector sequence. Preferably, the recombinant Koi herpesvirus comprises a BAC (bacterial artificial chromosome) vector sequence which is inserted into the Koi herpesvirus genome.

Further preferred, the recombinant Koi herpesvirus comprises a BAC (bacterial artificial chromosome) vector sequence which is inserted into the viral thymidine kinase gene, or alternatively any other viral gene which contributes to virulence and/or any other viral gene which is not essential for viral replication and/or any intergenic region. It is particularly preferred that the BAC vector sequence is flanked by sequences mediating homologous recombination, preferably *IoxP.* Further preferred, the BAC vector sequence comprises a selectable marker. Even further preferred, the selectable marker is a drug selectable marker. Even more preferred the selectable marker is a gene encoding green fluorescent protein. In a further preferred embodiment the genome of said recombinant herpesvirus is present in the form of a plasmid. This is achieved by isolating circular forms of the above mentioned recombinant Koi herpesvirus comprising a BAC vector sequence and introduction into bacterial cells. It should be noted that it is not essential for the invention that the BAC (bacterial artificial chromosome) vector sequence is inserted into one or more of the viral genes which contribute to virulence, as long as one or more of the mentioned genes which contribute to virulence is/are deficient by genetic engineering techniques. Therefore, the BAC vector sequence may be inserted into any intergenic region of the virus, if the one or more of the viral genes which contribute to virulence are At least viral thymidine kinase gene and/or viral ORF140: putative thymidylate kinase gene.

In an alternative embodiment of the present invention the BAC vector sequence is excised from the herpesvirus genome thereby leaving a heterologous sequence at the excision site or former insertion site in the herpesvirus genome. Preferably, the heterologous sequence has a size of less than 200 nucleotides. The excision is achieved by introduction of the recombinant KHV into a permissive eukaryotic cell expressing the Cre recombinase which is excising the *IoxP*-flanked BAC vector sequence. The resulting recombinant KHV may preferably be able to form infectious particles.

In further preferred embodiments of the recombinant Koi herpesvirus further viral genes are modified in their activity or deficient or deleted.

In a further preferred embodiment the recombinant Koi herpesvirus is further comprising a heterologous sequence or heterologous gene. By this any gene may be introduced into the respective inoculated fish. Preferably, the further heterologous gene is G glycoprotein of rhabdovirus causing carp spring viraemia. Such a construct will not only protect the vaccinated fish against KHV but also against carp spring viraemia.

The present invention also provides a recombinant Koi herpesvirus (KHV), which is deficient in thymidine kinase gene.

Further, the present invention provides a recombinant Koi herpesvirus (KHV), which is deficient in ORF140: putative thymidylate kinase gene.

Even further, the present invention provides a recombinant Koi herpesvirus, which is deficient in thymidine kinase gene and ORF140: putative thymidylate kinase gene.

Surprisingly, the recombinant Koi herpesvirus which is deficient in thymidine kinase gene results in a strongly reduced mortality of carps when infected with this herpesvirus and provides immunity against wild-type Koi herpesvirus. The recombinant Koi herpesvirus which is deficient in thymidine kinase gene and ORF140: putative thymidylate kinase gene, as well as the recombinant Koi herpesvirus which is deficient only in ORF140: putative thymidylate kinase gene show no mortality of carps when infected with this herpesvirus and provides immunity against wild-type Koi herpesvirus.

The present invention also provides a DNA sequence encoding recombinant KHV as defined in the claims for use in vaccine purposes and/or prophylactic health care in fish, wherein said DNA sequence which has the capability to confer immunity on fish, preferably on carps, more preferably on *Cyprinus carpio,* more preferred on *Cyprinus carpio carpio* or *Cyprinus carpio koi,* against a disease caused by Koi herpesvirus (KHV).

Said DNA sequence is comprising Koi herpesvirus genome or a part thereof. Whereby the Koi herpesvirus genome is deficient in at least one gene selected from the group consisting of thymidine kinase gene, ORF140: putative thymidylate kinase gene, or any combination thereof, preferably is deficient in the thymidine kinase gene and ORF140: putative thymidylate kinase gene.

In a further preferred embodiment the DNA sequence for use as a vaccine is further comprising a heterologous sequence or heterologous gene. By this any gene may be introduced into the respective inoculated fish. Preferably, the further heterologous gene is G glycoprotein of rhabdovirus causing carp spring viraemia.

The present invention also provides a Koi herpesvirus genome deficient in at least one gene selected from the group consisting of thymidine kinase gene and ORF140: putative thymidylate kinase gene, further preferred deficient in both genes, for use as a vector.

The recombinant Koi herpesvirus according to the present invention can be used in carp *(Cyprinus carpio)* and also in other fish species than carp as vector to transfer a gene of interest into such fish species or cells derived from them other than carp *(Cyprinus carpio).* The recombinant Koi herpesvirus according to the present invention can be used as an in vivo expression vector in all fish species that are sensitive to the infection by wild-type Koi herpesvirus, or by the recombinant Koi herpesvirus of the present invention. Preferably, such fish may be any of those which cohabit with carps. Further preferred these fish are selected from the group consisting of fish of the tilapia group (Oreochromis niloticus), silver perch (Bidyanus bidyanus), silver carp (Hypophthalmichthys molitrix), gold fish (Carassius auratus), crucian carp (Carassius carassius), golden ide (Leuciscus idus), sheatfish (Ictalurus nelos), and grass carp (Ctenopharyngodon idella). This means that the recombinant Koi herpesvirus (KHV) according to the present invention can be used as a vector to transfer a polynucleotide or gene of interest into carp *(Cyprinus carpio)* or cells derived from them or into fish species or cells derived from them other than carp *(Cyprinus carpio).* As outlined above said recombinant Koi herpesvirus (KHV) according to the present invention is deficient in at least one gene which contributes to virulence selected from the group consisting of thymidine kinase gene; ORF140: putative thymidylate kinase gene, or any combination thereof.

The recombinant KHV for use as a vector, may further comprise a heterologous sequence or heterologous gene. By this any gene may be introduced into the respective fish. Preferably, the further heterologous gene is G glycoprotein of rhabdovirus causing carp spring viraemia. Such a construct will not only protect the vaccinated fish against KHV but also against carp spring viraemia.

The recombinant KHV for use as a vector may further comprise a BAC vector sequence, may have the capability to replicate and and may have the capability also to reconstitute infectious particles when introduced into permissive eukaryotic cells.

The recombinant KHV for use as a vector is able to express KHV viral genes when introduced into permissive cells or fish individuals, in order to induce a protective immune response against a disease caused by Koi herpesvirus (KHV). Further preferred, the recombinant KHV for use as a vector has the capability to confer immunity on fish, preferably on carps, more preferably on *Cyprinus carpio,* more preferred on *Cyprinus carpio carpio* or *Cyprinus carpio Koi* against a disease caused by Koi herpesvirus (KHV).

The present invention also provides a cell which comprises the recombinant Koi herpesvirus or the DNA sequence or the vector according to the present invention as mentioned and characterized above. Preferably, the cell is a bacterial cell. In an alternative embodiment the cell is a permissive eukaryotic cell, preferably a cell derived from fish, preferably from carp, more preferred from *Cyprinus carpio carpio* or *Cyprinus carpio koi.* The cell, which may be either prokaryotic or eukaryotic, may be obtained by infection or transfection of the above mentioned recombinant Koi herpesvirus or the above mentioned DNA sequence or the above mentioned vector according to the present invention. The cell may be designated as "transformant". An *E*. *coli* strain comprising the recombinant Koi herpesvirus having a BAC vector sequence incorporated in the viral thymidine kinase gene was deposited at the Belgian Coordinated Collection of Microorganisms (BCCM) (BCCM/LMPB Plasmid Collection, Department of Molecular Biology, Ghent University, Technologiepark 927, 9052 Gent - Zwijnaarde, Belgium). The cell was deposited on July 13, 2007 and was assigned with the deposition number LMBP 5658. This cell is particularly preferred.

The present invention also provides a virus or viral particles produced by the cell according to the present invention.

The present invention further provides a method for the production of infectious particles of recombinant Koi herpesvirus (KHV), comprising the step of introduction of any one of the following recombinant Koi herpesvirus (KHV) genome or recombinant Cyprinid herpesvirus 3 (CyHV-3) genome into permissive eukaryotic cells: (a) a recombinant Koi herpesvirus genome (KHV) or recombinant Cyprinid herpesvirus 3 (CyHV-3) genome comprising a bacterial artificial chromosome (BAC) vector sequence; (b) a recombinant Koi herpesvirus genome (KHV) or recombinant Cyprinid herpesvirus 3 (CyHV-3) genome wherein the bacterial artificial chromosome (BAC) vector sequence is excised from the construct of (a); and culturing the host cell to produce recombinant Koi herpesvirus (KHV) or recombinant Cyprinid herpesvirus 3 (CyHV-3).

Further, the present invention is also directed to infectious particles generated by the above mentioned method. Further preferred the infectious particles provided are comprising any one of the following: (a) a recombinant Koi herpesvirus (KHV) genome or recombinant Cyprinid herpesvirus 3 (CyHV-3) genome comprising a bacterial artificial chromosome (BAC) vector sequence; (b) a recombinant Koi herpesvirus (KHV) genome or recombinant Cyprinid herpesvirus 3 (CyHV-3) genome wherein the bacterial artificial chromosome (BAC) vector sequence is excised from the construct of (a).

The above mentioned recombinant Koi herpesvirus, the above mentioned DNA sequence the above mentioned vector or the above mentioned infectious particles can be used for the immunization of fish, preferably Cyprinus carpio carpio or Cyprinus carpio Koi individuals by injection or balneation or per os. Therefore, the present invention also provides a Cyprinus carpio carpio or Cyprinus carpio Koi individual immunized with the above mentioned recombinant Koi herpesvirus or with the above mentioned DNA sequence or with the above mentioned vector or with the above mentioned infectious particles according to the present invention.

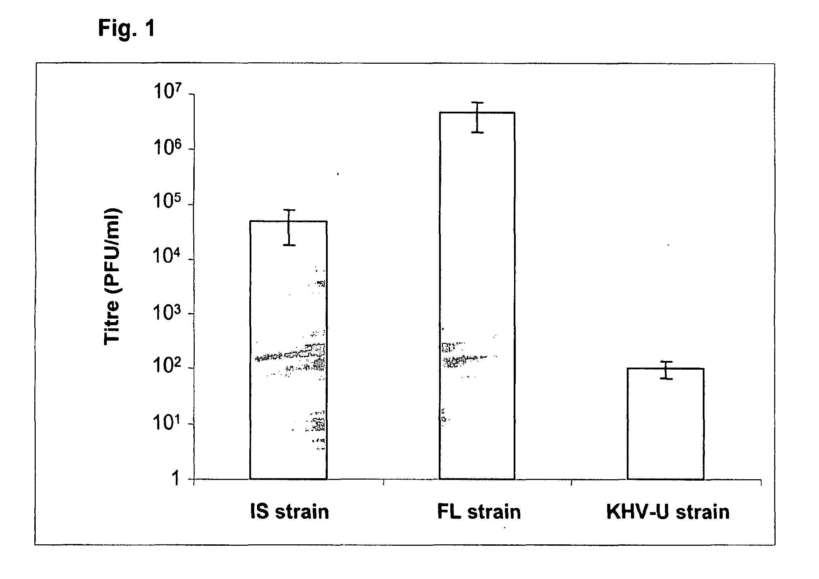

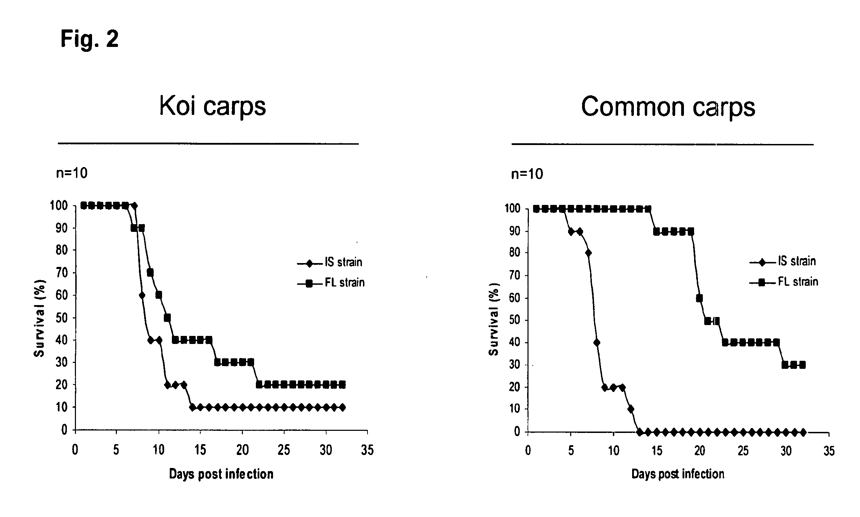

Also described is a Koi herpesvirus (KHV) or CyHV-3. This Koi herpesvirus isolate, herein also designated as FL strain, was deposited at the Belgian Coordinated Collection of Microorganisms (BCCM) (BCCM/LMPB Plasmid Collection, Department of Molecular Biology, Ghent University, Technologiepark 927, 9052 Gent - Zwijnaarde, Belgium). The FL isolate was deposited on July 13, 2007 and was assigned with the deposition number LMBP 6581 CB. This isolate (FL) shows three important properties: (i) it has very efficient growth properties in cell culture; (ii) it has a lower pathogenicity than comparable wild-type strains; and (iii) it shows a 2 weeks delay of the onset of the disease in 2 g common carps. In order to obtain such an isolate, different KHV field isolates were compared for their growth properties in vitro (Fig. 1) and their virulence in Koi (7 g) and common (2 g) carps (Fig. 2). Based on the results of these tests, a strain called hereafter FL was selected. Compared to a KHV/CyHV-3 reference strain (IS strain), the FL strain replicated more efficiently in vitro and was less pathogenic in vivo. When assayed on 7 g Koi carps, the IS and the FL strain led to 90% and 80% mortality, respectively (Fig. 2). When assayed on 2 g common carps, the onset of the disease induced by the FL strain was delayed by two weeks in comparison to the IS strain. In view of the observed delay by two weeks compared to the higher virulent KHV IS strain and considering the fact that in common carps the onset of the disease caused by the higher virulent KHV IS strain occurs after about 1 week, the FL strain as such is considered to represent a suitable parental strain for the development of live attenuated recombinant strains.

The KHV FL strain as described (deposition number LMBP 6581 CB) may be very useful for the production of an inactivated vaccine and therefore will have a significant industrial potential.

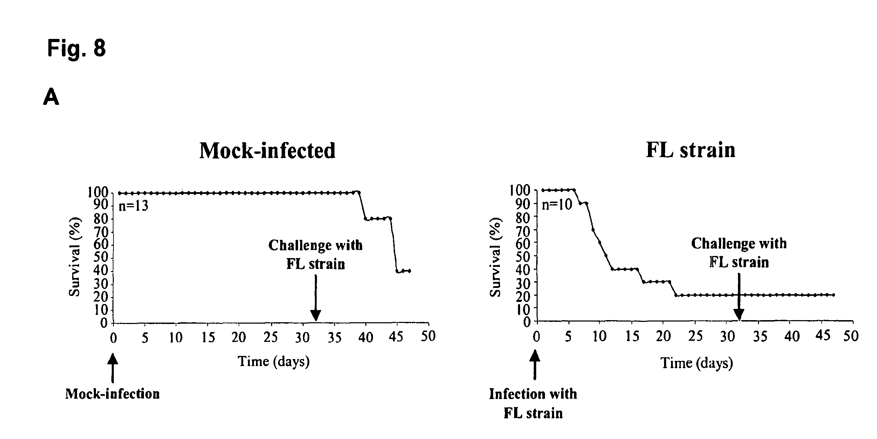

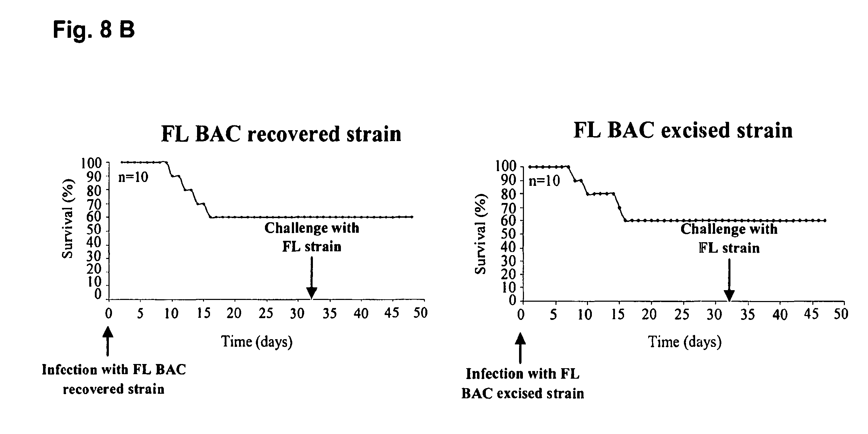

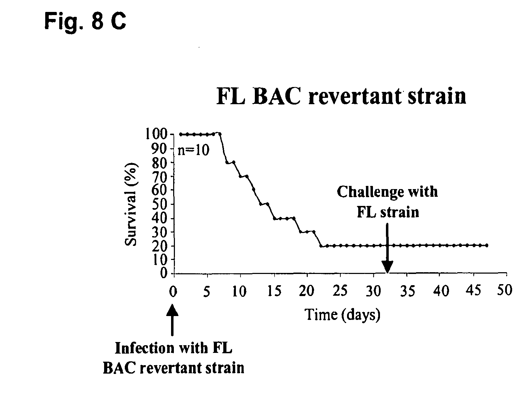

The FL isolate was used to construct the above mentioned recombinant Koi herpesvirus according to the present invention. For example, a BAC vector sequence was introduced into the viral thymidine kinase gene, in order to obtain a recombinant Koi herpesvirus according to the invention. However, any other KHV isolate can be used in order to construct a recombinant Koi herpesvirus according to the present invention. A FL BAC excised strain was assayed as a potential parental vaccine strain on Koi carps (see **Figure 8**). 7 g Koi carps were inoculated with the FL strain, the FL BAC recovered strain and the FL BAC excised strain leading to 80%, 40% and 40% mortality, respectively. This experiment demonstrated that the disruption of the TK locus contribute to a drastic attenuation of the virus. Independently of the strain used for the primary inoculation, all fishes that survived to the primo-inoculation developed a protective immune response against a further challenge with the virulent FL strain (see arrow "challenge" in **Figure 8**). Altogether these data support the potential of the FL BAC clone as a parental plasmid for the development of multi-attenuated recombinant vaccines.

A derivative of the Koi herpesvirus (KHV) deposition number LMBP 6581CB may be deficient in one or more viral genes which is/are not essential for replication. A derivative of the Koi herpesvirus (KHV) deposition number LMBP 6581CB may be deficient in at least one gene which contributes to virulence. The derivative of the Koi herpesvirus (KHV) deposition number LMBP 6581CB according to the present invention is deficient in at least one gene which contributes to virulence selected from the group consisting of thymidine kinase gene; ORF140: putative thymidylate kinase gene, or any combination thereof. It is further preferred that the derivative of the Koi herpesvirus (KHV) deposition number LMBP 6581CB according to the present invention is deficient in viral thymidine kinase gene and at least one further gene which contributes to virulence selected from the group consisting of ORF12: putative tumor necrosis factor (TNF) receptor gene; ORF 16: putative G-protein coupled receptor (GPCR) gene; ORF 134: putative Interleukin 10 homologue gene; ORF140: putative thymidylate kinase gene, or any combination thereof. It is further particularly preferred that the derivative of the Koi herpesvirus (KHV) deposition number LMBP 6581CB is deficient in all of above mentioned genes. Moreover, the derivative of the Koi herpesvirus (KHV) deposition number LMBP 6581 CB may be deficient in further viral genes. Preferably, a derivative of the Koi herpesvirus (KHV) deposition number LMBP 6581CB is deficient in thymidine kinase gene and ORF140: putative thymidylate kinase gene.

In a further preferred embodiment of the present invention the derivative of the Koi herpesvirus (KHV) deposition number LMBP 6581 CB is in a live form. Preferably, the derivative of the Koi herpesvirus (KHV) deposition number LMBP 6581CB has the capability to reconstitute infectious particles when introduced into permissive eukaryotic cells or fish individuals preferably in carps, more preferably in *Cyprinus carpio,* even further preferred in *Cyprinus carpio carpio* and/or *Cyprinus carpio koi.*

In a further preferred embodiment of the present invention the derivative of the Koi herpesvirus (KHV) deposition number LMBP 6581CB as defined in the claims results in a mortality rate of 40 % or less, preferably of 20 % or less, more preferred of 10 % or less, further preferred of 1% or less, further preferred 0.1 % or less and most preferred will result in a mortality rate of 0.0% in fish, preferably in carps, more preferred *Cyprinus carpio carpio* or *Cyprinus carpio Koi* infected with said herpesvirus.

In an alternative embodiment of the present invention the derivative of the Koi herpesvirus (KHV) deposition number LMBP 6581CB as defined by the claims is in inactivated and non-replicative form or in deficient attenuated form.

The present invention also provides a vaccine providing immunity against a disease caused by Koi herpesvirus (KHV) or CyHV-3.

Further preferred the vaccine is comprising the above mentioned recombinant Koi herpesvirus or the above mentioned DNA sequence or the above mentioned vector or the above mentioned infectious particles or a derivative of the Koi herpesvirus (KHV) or CyHV-3 deposition number LMBP 6581 CB according to the present invention.

In a further preferred embodiment the above mentioned vaccine is used providing immunity against a disease caused by Koi herpesvirus (KHV) or CyHV-3.

The present invention also provides the use of the above mentioned recombinant Koi herpesvirus or of the above mentioned DNA sequence or of the above mentioned vector or of the above mentioned infectious particles or a the above mentioned derivative of Koi herpesvirus (KHV) or CyHV-3 deposition number LMBP 6581 CB for the manufacture of a medicament/vaccine for providing immunity against a disease caused by Koi herpesvirus (KHV).

The vaccine according to the present invention is useful for providing immunity against a disease caused by Koi herpesvirus (KHV) or CyHV-3. Any type of vaccine may be produced e.g. inactivated vaccine, inactivated and non-replicative vaccine, recombinant attenuated vaccine, recombinant attenuated vaccine expressing a foreign transgene, DNA vaccine etc. The preferred routes of administration of said vaccine are *per os,* balneation and injection but other routes are not excluded.

The present invention also provides a vaccine providing immunity against a disease caused by rhabdovirus causing carp spring viraemia, which vaccine is comprising the recombinant Koi herpesvirus, or the DNA sequence or the vector, wherein each of which is further comprising a heterologous sequence or heterologous gene from said rhabdovirus, preferably the G glycoprotein of said rhabdovirus causing carp spring viraemia.

As used herein, the term "vaccine" refers to a substance capable of establishing or improving immunity of a host to a particular disease. In cases where a disease is caused by a pathogen, such vaccine substance generally is constituted of life attenuated or inactivated pathogen or parts thereof. Such vaccine may produce immunity prophylactically or therapeutically.

Typically, vaccines are prepared as liquid solutions, emulsions or suspensions for injection or delivery in water. For instance, a liquid emulsion or emulsifiable concentrate can be prepared in order to be added to a water tank or bath where the fish are held. Solid (e.g. powder) forms suitable for dissolution in, or suspension in, liquid vehicles or for mixing with solid food, prior to administration may also be prepared. The vaccine may be a lyophilised culture in a ready to use form for reconstitution with a sterile diluent. For instance, lyophilized cells may be reconstituted in 0.9% saline (optionally provided as part of the packaged vaccine product). A preferred formulation of injectable vaccine is an emulsion. Liquid or reconstituted forms of the vaccine may be diluted in a small volume of water (e.g. 1 to 100 volumes) before addition to a pen, tank or bath.

In one preferred embodiment, the vaccine preparation comprising the above mentioned recombinant KHV or a derivative of the KHV FL strain is in a dry form, e. g. in a powder form, lyophilized, in a compressed pellet or tablet form, etc. In another embodiment, said virus may be in the form of a tissue culture fluid. Said fluid may be stored at the ambience, preferably at -70 °C, most preferably as a solution containing glycerol. In one specific example, the tissue culture fluid contains 20% glycerol.

The recombinant KHV or a derivative of the KHV FL strain disclosed in the present invention may be converted into a dry form by a number of methods. A particularly preferred form of drying is through lyophilization. Prior to drying, e.g. lyophilization procedure, a variety of ingredients may be added to the medium such as preservatives, anti-oxidants or reducing agents, a variety of excipients, etc. Such excipients may also be added to the dry, e. g. lyophilized active-attenuated virus also after the drying step.

Therefore, in a preferred embodiment, the vaccine according to the invention is in a freeze-dried form. To reconstitute a freeze-dried composition, it is suspended in a physiologically acceptable diluent. Such a diluent can e.g. be as simple as sterile water, or a physiological salt solution. In a more complex form the freeze-dried vaccine may be suspended in an emulsion e.g. as described in EP 1,140,152.

Immunization is typically performed by adding the inactivated virus, inactivated and non-replicative virus, deficient attenuated virus, recombinant attenuated virus, recombinant attenuated virus expressing a foreign transgene, an avirulent virus form such as live-attenuated virus, DNA vaccine to the water containing the fish to be immunized. It is also possible to inject the vaccine preparation directly into the fish. In order to boost the immune response, the preparation may also include a variety of adjuvants, cytokines or other immunostimulants, particularly in the case of preparations that are intended for injection. The vaccine preparation, particularly where it is introduced into the water containing the fish, may also include a variety of stabilizers, antibiotics, etc.

The vaccine further may comprise an immune modulator adjuvant, stabilizer, antibiotics, immunostimulants or other substances. An adjuvant is an immunostimulatory substance boosting the immune response of the host in a non-specific manner. These adjuvants are known from those already used in human and veterinary medicine. The adjuvant may be hydrophilic adjuvants, e.g. , aluminum hydroxide or aluminum phosphate, or hydrophobic adjuvants, e.g. mineral oil based adjuvants. Adjuvants such as muramyl dipeptides, avridine, aluminium hydroxide, aluminium phosphate, oils, oil emulsions, saponins, dextran sulphate, glucans, cytokines, block co-polymers, immunostimulatory oligonucleotides and others known in the art may be admixed with the inactivated viral supernatant. A preferred adjuvant is Freund's Incomplete Adjuvant (FIA). The amount of adjuvant added depends on the nature of the adjuvant itself. FIA may advantageously be emulsified with inactivated viral supernatant in a ratio of about 1:1 by volume.

Examples of adjuvants frequently used in fish farming are muramyldipeptides, lipopolysaccharides, several glucans and glycans and Carbopol® (a homopolymer). Suitable adjuvants are e.g. water in oil (w/o) emulsions, o/w emulsions and w/o/w double-emulsions. Oil adjuvants suitable for use in w/o emulsions are e.g. mineral oils or metabolisable oils. Mineral oils are e.g. Bayol®, Marcol® and Drakeol®; metabolisable oils are e.g. vegetable oils, such as peanut oil and soybean oil, or animal oils such as the fish oils squalane and squalene. Alternatively a vitamin E (tocopherol) solubilisate as described in EP 382,271 may advantageously be used. Very suitable o/w emulsions are e.g. obtained starting from 5-50 % w/w water phase and 95-50 % w/w oil adjuvant, more preferably 20-50 % w/w water phase and 80-50 % w/w oil adjuvant are used. The amount of adjuvant added depends on the nature of the adjuvant itself, and information with respect to such amounts provided by the manufacturer.

The vaccine according to the invention additionally may comprise a stabilizer. A stabilizer can be added to a vaccine according to the invention e.g. to protect it from degradation, to enhance the shelf-life, or to improve freeze-drying efficiency. Useful stabilizers are i.a. SPGA (Bovarnik et al., 1950, J. Bacteriology, vol. 59, p. 509), skimmed milk, gelatine, bovine serum albumin, carbohydrates e.g. sorbitol, mannitol, trehalose, starch, sucrose, dextran or glucose, lactoses, proteins such as albumin or casein or degradation products thereof, and buffers, such as alkali metal phosphates.

Antibiotics such as neomycin and streptomycin may be added to prevent the potential growth of germs. The immunostimulants may enhance protection afforded by vaccines and provide non-specific protection against a number of diseases. Many chemicals and other substances have been reported as having immunostimulating properties in fish, i.e., chemicals and glucans, extracts from algae-algines (alginic acid silylic esters) and cytokines.

In addition, the vaccine may comprise one or more suitable surface-active compounds or emulsifiers, e.g. Span ® or Tween®. The vaccine may also comprise a so-called "vehicle". A vehicle is a compound to which the KHV virus (either in form of a virus particle or in form of DNA) according to the invention adheres, without being covalently bound to it. Such vehicles are i.a. bio-microcapsules, micro-alginates, liposomes and macrosols, all known in the art. A special form of such a vehicle is an Iscom, described above. It goes without saying that admixing other stabilizers, carriers, diluents, emulsions, and the like to vaccines according to the invention are also within the scope of the invention. Such additives are for instance described in well-known handbooks such as: "Remington: the science and practice of pharmacy" (2000, Lippincot, USA, ISBN: 683306472), and: "Veterinary vaccinology" (P. Pastoret et al. ed., 1997, Elsevier, Amsterdam, ISBN: 0444819681).

The recombinant KHV or a derivative of the KHV FL strain when used in its dry form in a vaccine may further include a reconstitution fluid, preferably sterile water, saline or physiological solution. It may also contain small amounts of residual materials from the manufacturing process such as cell proteins, DNA, RNA, etc. While these materials are not additives per se, they may nonetheless be present in the vaccine formulation.

The invention further relates to a vaccine formulation comprising the recombinant KHV or a derivative of the KHV FL strain according to the present invention for use in a method for immunizing fish against the viral infection caused by KHV described hereinbefore, said method comprising administration to susceptible fish, said vaccine being administered in an amount sufficient to induce immunity to subsequent infection by KHV.

The vaccine may be administered to the aquaculture fish individually-orally, e.g. through their feed or by forced oral administration, or by injection (intramuscular or the intraperitoneal route). Alternatively the vaccine may be administered simultaneously to the entire fish population contained in a body of water by spraying, dissolving and/or immersing the vaccine. These methods are useful for vaccination of all kinds of fish, e.g., food and ornamental, and in various environments such as, and not limited to, ponds, aquariums, natural habitat and fresh water reservoirs.

A further aspect of the invention relates to a DNA vaccine comprising the recombinant KHV according to the invention. Nucleic acid vaccines (or gene- or genetic-vaccines as they are called) may require a targeting- or a delivery vehicle to target or protect it, or to assist in its uptake by (the cells of) the host. Such vehicles may be biologic or synthetic, and are for instance bacteriophages, virus-like particles, liposomes, or micro-, powder-, or nano particles.

DNA vaccines can easily be administered through intradermal application e.g. using a needle-less injector such as a GeneGun®. This way of administration delivers the DNA directly into the cells of the animal to be vaccinated. A preferred amount of a nucleic acid, a DNA fragment, or a recombinant DNA molecule according to the invention, comprised in a pharmaceutical composition according to the invention (as outlined below) is in the range between 10 pg and 1000 µg. Preferably, amounts in the range between 0.1 and 100 µg are used. Alternatively, fish can be immersed in solutions comprising e.g. between 10 pg and 1000 µg/ml of the DNA to be administered. All these are well-known in the art.

Similarly, a targeting- or delivery vehicle comprising a nucleic acid, a DNA fragment, or a recombinant DNA molecule according to the invention, is within the scope of a carrier according to the invention. These uses will result in nucleic acid being delivered, or protein being expressed inside the target organism or its cells.

In a further aspect, the invention relates to a vaccine according to the invention, for use in a method of vaccination of fish by administering said vaccine to such organism in a pharmaceutically effective amount and in a pharmaceutically acceptable carrier. A "pharmaceutically effective amount" is described in detail below.

A vaccine according to the invention may take any form that is suitable for administration in the context of aqua-culture farming, and that matches the desired route of application and desired effect. Preparation of a vaccine according to the invention is carried out by means conventional for the skilled person.

Preferably the vaccine according to the invention is formulated in a form suitable for injection or for immersion vaccination, such as a suspension, solution, dispersion, emulsion, and the like. Commonly such vaccines are prepared sterile.

The dosing scheme of the application of a vaccine according to the invention to the target organism can be in single or multiple doses, which may be given at the same time or sequentially, in a manner compatible with the dosage and formulation and in such an amount as will be immunologically effective. It is well within the capacity of the skilled person to determine whether a treatment is "immunologically effective", for instance by administering an experimental challenge infection to vaccinated animals, and next determining a target animals' clinical signs of disease, serological parameters, or by measuring reisolation of the pathogen.

What constitutes a "pharmaceutically effective amount" for a vaccine according to the invention that is based upon a nucleic acid according to the invention is dependent on the desired effect and on the target organism. Determination of the effective amount is well within the skills of the routine practitioner. A preferred amount of a nucleic acid, a DNA fragment, or a recombinant DNA molecule according to the invention, comprised in a pharmaceutical composition according to the invention, has been described above.

A preferred amount of a vaccine comprising recombinant KHV virus or a derivative of the KHV FL strain according to the invention is expressed for instance as plaque forming units (pfu), colony forming units (cfu) or tissue culture infective dose 50% (TCID50). For instance for a live viral vector a dose range between 1 and 10¹⁰ plaque forming units (pfu) per animal dose may advantageously be used; preferably a range between 10² and 10⁶ pfu/dose. A suitable dosage range of virus is from about 10² to 10⁹ TCID50 (tissue culture infectious dose affecting 50% of cultures inoculated) per unit dose, preferably about 10⁴ to 10⁸ TCID50 per unit dose, more preferably about 10⁶ to 10⁷ TCID50 per unit dose, most preferably about 10⁷ TCID50 per unit dose. Preferably a single dosage unit is administered to the fish to be treated. Many ways of administration can be applied, all known in the art. The vaccines according to the invention are preferably administered to the fish via injection (intramuscular or the intraperitoneal route), immersion, dipping or per os. The protocol for the administration can be optimized in accordance with standard vaccination practice.

Preferably the vaccine is administered via immersion or per os. This is especially efficient in case of the use of such vaccines in the setting of commercial aqua-culture farming.

The vaccines according to the invention, described above, contribute to active vaccination, i.e. they trigger the host defense system.

### Modes for carrying out the invention

The present invention provides a recombinant Koi herpesvirus (KHV), which preferably is immunogenic in fish, preferably in carps, more preferably in *Cyprinus carpio,* more preferred in *Cyprinus carpio carpio* or *Cyprinus carpio koi.* In one embodiment of the invention the recombinant Koi herpesvirus has the capability to reconstitute infectious particles when introduced into permissive eukaryotic cells. The inventors of the present invention achieved the cloning of the KHV genome as a stable and infectious BAC clone. This goal was achieved by insertion of a modified *loxP-*flanked BAC cassette into the thymidine kinase locus. This insertion allowed the production of a KHV BAC clone stably maintained in bacteria and able to regenerate virions when transfected into permissive cells. However, the BAC (bacterial artificial chromosome) vector sequence may be inserted into the genes which contribute to virulence that are selected from the group consisting of thymidine kinase gene; ORF140: putative thymidylate kinase gene, or any combination thereof. Further preferred the recombinant KHV is deficient in both genes thymidine kinase gene and ORF140: putative thymidylate kinase gene.

Further, the *Iox*P-flanked BAC cassette can be excised from the genome of reconstituted virions by growing them on permissive cells expressing Cre recombinase. The present disclosure therefore is the first to describe the BAC cloning of KHV genome and is the first to provide the BAC cloning of a herpesvirus genome as large as KHV. The availability of the KHV BAC is an important advance that will allow the identification of viral genes involved in KHV pathogenesis, as well as the production of attenuated recombinant candidate vaccines.

The present invention was achieved by cloning of the KHV genome by the insertion of a modified *IoxP*-flanked BAC cassette into the thymidine kinase (TK) locus which was postulated to be non-essential for KHV replication. This insertion led to a BAC recombinant virus whose genome was stably maintained in bacteria and was able to regenerate virions when transfected into permissive cells.

A central advantage of the BAC cloning system over cosmid vectors used in prior art is that the F-plasmid is present in only one or a maximum of two copies per cell reducing the potential for recombination between DNA fragments and, more importantly, avoiding the lethal overexpression of cloned bacterial genes. However, the presence of the BAC as just a single copy means that plasmid DNA has to be extracted from a large volume of culture to obtain sufficient DNA for sequencing and it is described here in the examples as simplified protocol to achieve this.

The term "BAC vector" refers to a plasmid which is produced using F plasmid of *E*. *coli* and a vector which can stably maintain and grow a large size DNA fragment of about 300 kb or more in bacteria, such as *E*. *coli* and the like. The BAC vector contains at least a region essential for the replication of the BAC vector. Examples of such a region essential for replication include, but are not limited to, the origin of replication of F plasmid and variants thereof.

As used herein, the term "BAC vector sequence" refers to a sequence comprising a sequence essential for the function of a BAC vector. Optionally, the BAC vector sequence may further comprise a "recombination protein-dependent recombinant sequence" and/or a "selectable marker".

As used herein, the term "homologous recombination" indicates that two different homologous nucleic acid molecules encounter each other, crossover occurs, and a new combination of nucleic acid is generated. As used herein, the term "sequence mediating homologous recombination" refers to a sequence which causes homologous recombination which is dependent from a specific recombination protein, which is catalyzing, carrying out or assisting in homologous recombination. Such a recombination protein preferably acts specifically on a "sequence mediating homologous recombination" and does not act on other sequences.

Examples of representative pairs of such a sequence and recombination protein catalyzing, carrying out or assisting homologous recombination include, but are not limited to: a combination of a bacteriophage P1-derived *IoxP* (locus of crossover of P1) sequence and a Cre (cyclization recombination) protein, a combination of Flp protein and FRT site, a combination of C31 and attB or attP, a combination of resolvase and res site.

As used herein, the term "selectable marker" refers to a gene which functions as an index for selection of a host cell containing a BAC vector. Examples of a selectable marker include, but are not limited to, fluorescent markers, luminiscent markers, and drug selectable markers. An example of a "fluorescent marker" is, but is not limited to, a gene encoding a fluorescent protein, such as a green fluorescent protein (GFP). An example of a "luminiscent marker" is, but is not limited to, a gene encoding a luminescent protein, such as luciferase. An example of a "drug selectable marker" is, but is not limited to, a gene encoding a protein selected from the group consisting of: dihydrofolate reductase gene, glutamine synthase gene, aspartic acid transaminase, metallothionein (MT), adenosine deaminase (ADA), adenosine deaminase (AMPD1, 2), xanthine-guanine-phosphoribosyl transferase, UMP synthase, P-glycoprotein, asparagine synthase, and ornithine decarboxylase. Examples of a combination of a drug selectable marker and a drug include: a combination of dihydrofolate reductase gene (DHFR) and methotrexate (MTX), a combination of glutamine synthase (GS) gene and methionine sulfoximine (Msx), a combination of aspartic acid transaminase (AST) gene and N-phosphonacetyl-L-aspartate) (PALA), a combination of MT gene and cadmium (Cd²⁺), a combination of adenosine deaminase (ADA) gene and adenosine, alanosine, or 2'-deoxycoformycin, a combination of adenosine deaminase (AMPD1, 2) gene and adenine, azaserine, or coformycin, a combination of xanthine-guanine-phosphoribosyltransferase gene and mycophenolic acid, a combination of UMP synthase gene and 6-azaulysine or pyrazofuran, a combination of P-glycoprotein (P-gp, MDR) gene and multiple drugs, a combination of asparagine synthase (AS) gene and -aspartyl hydroxamic acid or albizziin, and a combination of ornithine decarboxylase (ODC) gene and - difluoromethyl-ornithine (DFMO).

Conveniently, the BAC cassette as used herein further contains a gene which confers resistance to chloramphenicol (CmR), a selection marker in prokaryotic cells. Further comprised in the BAC cassette is a resistance gene for G418 (NeoR) which is in fusion with EGFP (EGFP-Neo). As result, this fusion protein between EGFP and Neo confers EGFP fluorescence and resistance to G418. The EGFP-Neo gene is under the control of the HCMV IE promoter and represents a selection marker in eukaryotic cells.

As used herein, the term "vector" refers to a vehicle made of a polynucleotide or which contains a polynucleotide which can transfer a polynucleotide sequence or gene of interest to a target cell. The vector may be a "viral vector" or a "plasmid vector" or may combine both properties in one construct. Examples of a vector include vectors which are capable of self replication or capable of being incorporated into a chromosome within host cells (e.g., prokaryotic cells, yeast, animal cells, plant cells, insect cells, whole animals, and whole plants), and contain a promoter at a site suitable for transcription of a polynucleotide or gene.

As used herein, the term "expression vector" refers to a nucleic acid sequence comprising a structural gene and a promoter for regulating expression thereof, and in addition, various regulatory elements in a state that allows them to operate within host cells. The regulatory element may include, preferably, terminators, selectable markers such as drug-resistance genes (e.g., a kanamycin resistance gene, a hygromycin resistance gene, etc.), and enhancers. It is well known to those skilled in the art that the type of an organism, expression vector and the type of a regulatory element may vary depending on the host cell.

As used herein, the term "promoter" refers to a base sequence which determines the initiation site of transcription of a gene and is a DNA region which directly regulates the frequency of transcription. Transcription is started by RNA polymerase binding to a promoter.

As used herein, the terms "transformation", "transduction" and "transfection" are used interchangeably unless otherwise mentioned, and refers to introduction of a nucleic acid into host cells. As a transformation method, any technique for introducing DNA into host cells can be used, including various well-known techniques, such as, for example, the electroporation method, the particle gun method (gene gun), the calcium phosphate method, and the like.

As used herein, the term "transformant" refers to the whole or a part of an organism, such as a cell, which is produced by transformation. Examples of a transformant include prokaryotic cells, yeast, animal cells, plant cells, insect cells and the like. Transformants may be referred to as transformed cells, transformed tissue, transformed hosts, or the like, depending on the subject. As used herein, all of the forms are encompassed, however, a particular form may be specified in a particular context.

As used herein the term "TK" refers to thymidine kinase gene and the term "TMPK" refers to ORF140: putative thymidylate kinase gene. The indication "TK⁻" refers to the situation that thymidine kinase gene is deficient. The indication "TK⁺" refers to the situation that thymidine kinase gene is functional. The indication "TMPK⁻" refers to the situation that putative thymidylate kinase gene is deficient. The indication "TMPK⁺" refers to the situation that putative thymidylate kinase gene is functional.

### Description of the figures

**Figure 1** shows the *in vitro* comparison of the growth properties of three KHV field isolates. CCB cells (Cyprinus carpio brain cells) were infected with the indicated KHV field isolate (Multiplicity of infection of 0.5 PFU/cell). After an incubation period of 2h, cells were washed with PBS and then overlaid with culture medium as described in the materials and methods. Five days post infection, supernatants of infected cultures were harvested and the amount of infectious virus was determined by plaque assay on CCB.

**Figure 2** shows the comparison of the pathogenicity of the KHV IS strain versus the KHV FL strain. On day 0, 7 g Koi carps (n=10) and 2 g common carps (n=10) were infected with either the IS strain (◆) or the FL strain (■) as described in the materials and methods (induction of KHV disease in fish). Percentage of survivors is expressed according to days post infection.

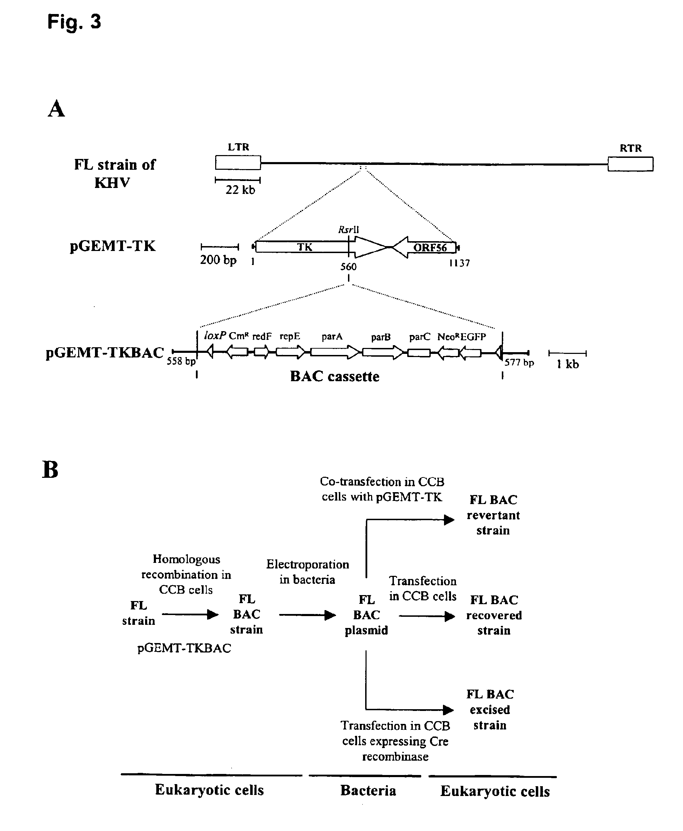

**Figure 3** is a schematic representation of the strategies used to produce infectious KHV FL BAC plasmid. (A) The genome of the KHV FL strain, flanked by two direct terminal repeats (LTR and RTR) is shown at the top. A *IoxP-*flanked BAC cassette was inserted into the *Rsr*II sites of TK (thymidine kinase) ORF of the pGEMT-TK vector resulting in pGEMT-TKBAC. In the latter vector, the BAC cassette is flanked by KHV homologous region of 558 bp and 577 bp. (CmR: confers resistance to chloramphenicol. This gene is under the control of a prokaryotic promotor. RedF: site-specific recombinase gene (it is a truncated version and whether the product of this gene is functional is not known); repE: mediates assembly of a replication complex at Ori2; parA, parB, parC: these three elements insure that the BAC remains as a single copy per bacterial cell; NeoR: resistance gene for G418 (fusion with EGFP); EGFP-Neo: This ORF encodes a fusion protein between EGFP and Neo. The expression product of this ORF confers EGFP fluorescence and resistance to G418. This ORF is under the control of the HCMV IE promotor. This gene is a selection marker in eukaryotic cells. **(B)** Flowchart of stages performed to produce KHV FL BAC plasmid, to control its infectivity (FL BAC recovered strain), to demonstrate the possibility of removing the *IoxP*-flanked BAC cassette from the genome of reconstituted virus (FL BAC excised strain) and to produce a wild type revertant strain derived from the FL BAC plasmid.

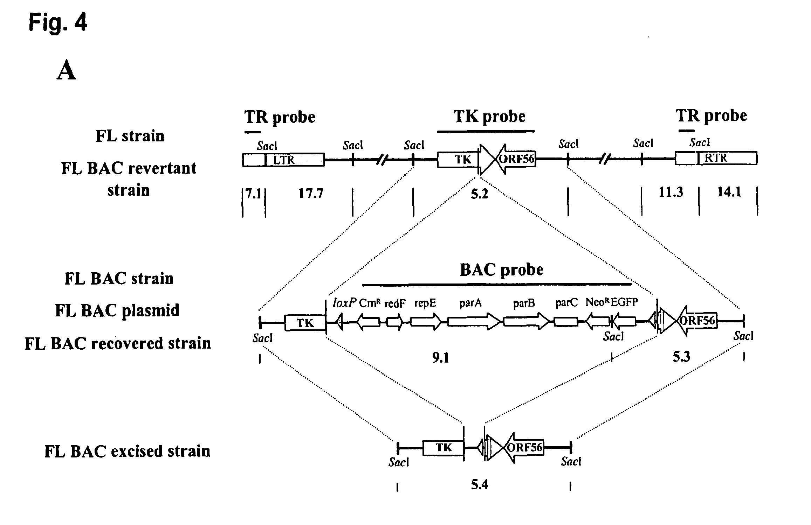

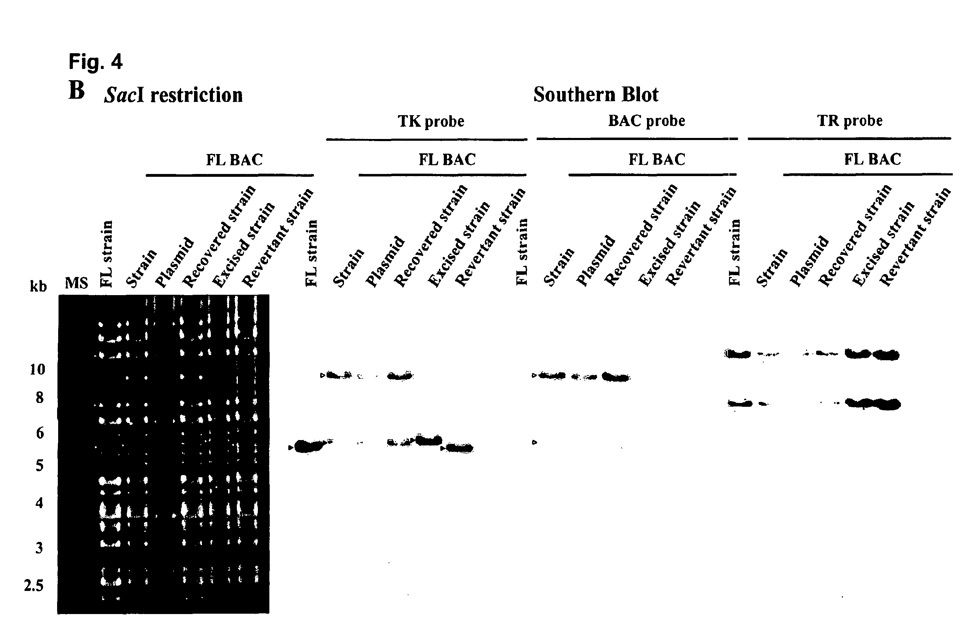

**Figure 4** shows structural analysis of KHV FL BAC plasmid and derived strains. **(A)** Schematic representation of some of the fragments generated by *Sac*I enzymatic restriction. The genome of the KHV FL and KHV FL BAC revertant strain is shown at the top. TK, BAC and TR probes are indicated by horizontal bold line. Fragments size in kb is indicated. Note that this cartoon is not drawn to scale. **(B)** The KHV FL BAC plasmid and the genome of the KHV FL, FL BAC, FL BAC recovered, FL BAC excised and FL BAC revertant strains were analyzed by *Sac*I restriction (left panel) and further tested by Southern blot using probes corresponding to TK ORF (second panel), the BAC cassette (third panel) or the TRs (fourth panel). Black/white arrows and open arrows indicate restriction fragments containing the TK ORF and the BAC cassette, respectively. Grey arrows indicate restriction fragments hybridizing with the TR probe. Markers sizes (MS) in kb are indicated on the left.

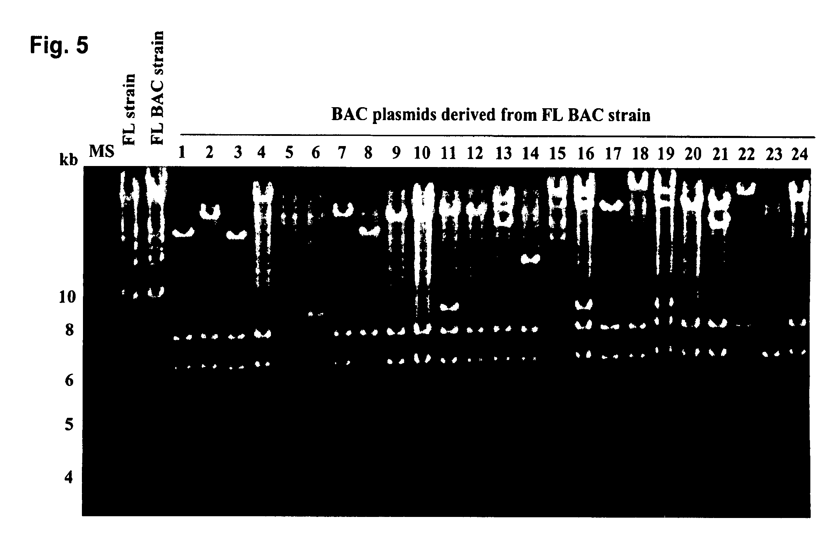

**Figure 5** shows the heterogeneity among KHV FL BAC plasmids. BAC plasmids were isolated from twenty-four independent *E*. *coli* clones and analyzed by *Hind*III. KHV parental (FL strain) and FL BAC strains were used as controls. Markers sizes (MS) in kb are indicated on the left.

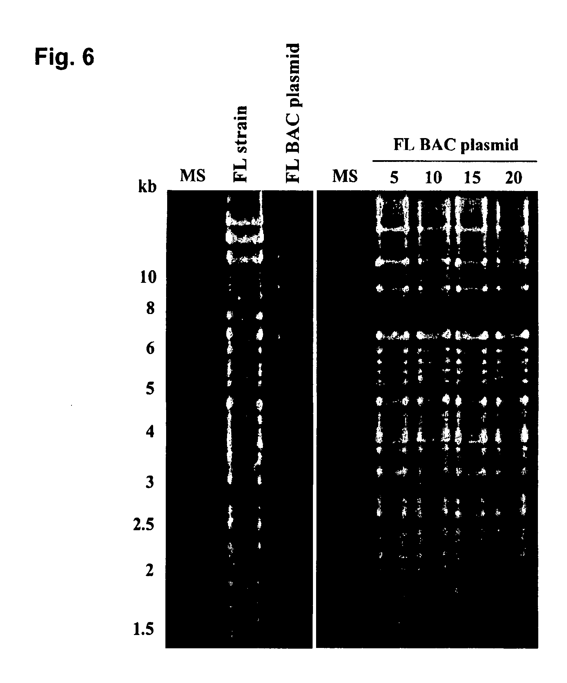

**Figure 6** shows the experiments on the stability of the KHV FL BAC plasmid in *E*. *coli.* DH10B cells containing KHV FL BAC plasmid were serially cultured for 20 consecutive days. At the indicated days, BAC DNAs were prepared as described in materials and methods then digested by *Sac*I. The KHV parental strain (FL strain) was used as control. Markers sizes (MS) in kb are indicated on the left.

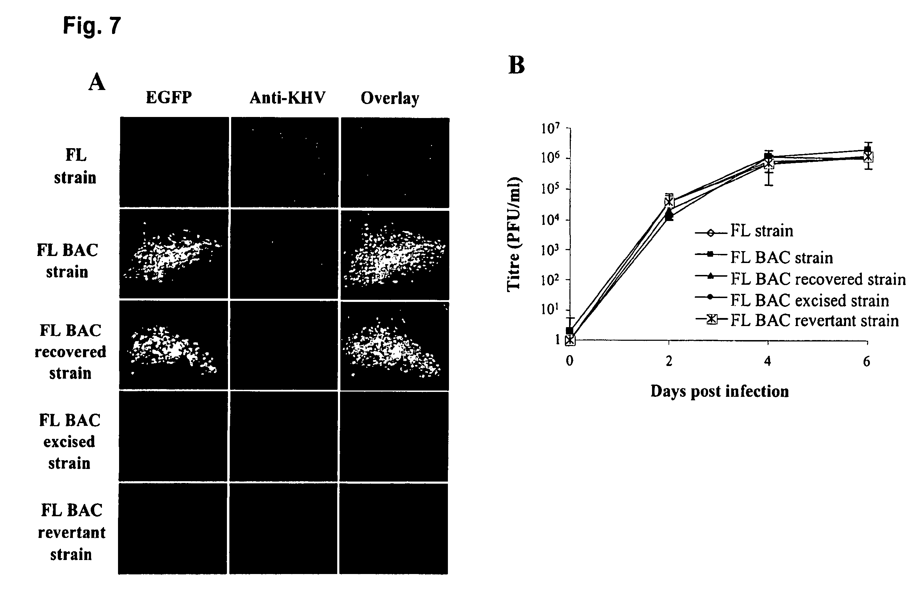

**Figure 7** shows the characterization of KHV FL derived strains. **(A)** Epifluorescent analysis of viral syncitia. CCB cells were infected (MOI of 0.1 PFU/cell) with KHV FL (i-iii), FL BAC (iv-iv), FL BAC recovered (vii-ix), FL BAC excised (x-xii) and FL BAC revertant (xiii-xv) strains and overlaid with DMEM containing 10 % FCS and 0.6 % (w/v) carboxymethylcellulose (Sigma) to obtain isolated syncitia. Seven days pi, syncitia were revealed by indirect immunofluorescent staining using Mab 8G12 and GAM Alexa 568 as the primary and secondary antibodies, respectively. The sets of three horizontal panels represent analyses of the same syncitium. Panels (i), (iv), (vii), (x), (xiii) and panels (ii), (v), (viii), (xi), (xiv) were analyzed for EGFP and GAM 568 fluorescent emissions, respectively. The merged EGFP and Alexa 568 signals are shown in panels (iii), (vi), (ix), (xii) and (xv). The side of each panel corresponds to 200 µm. **(B)** Replication kinetics of KHV recombinant strains were compared with those of the parental KHV FL strain as described in materials and methods. The data presented are the means of triplicate measurements.

**Figure 8** shows the cumulative incidence of survival rate of carps infected by FL BAC plasmid derived strains. On day 0, five groups, each consisting of 10 Koi carps (7 g) (with exception of mock infected groups consisting of 13 carps), were inoculated by IP injection with mock-infected culture medium **(A)** and culture medium containing 3 x 10² PFU of FL (TK⁺) **(A),** FL BAC recovered (TK⁻) **(B)**, FL BAC excised (TK⁻) **(B)** and FL BAC revertant strains (TK⁺) **(C)** as described in the material and methods. On day 32 post-infection, surviving fishes were challenged by IP injection with the parental FL strain (3 x 10² PFU/fish). Percentage of survival is expressed according to days post-infection. The results presented are representative of three independent experiments.

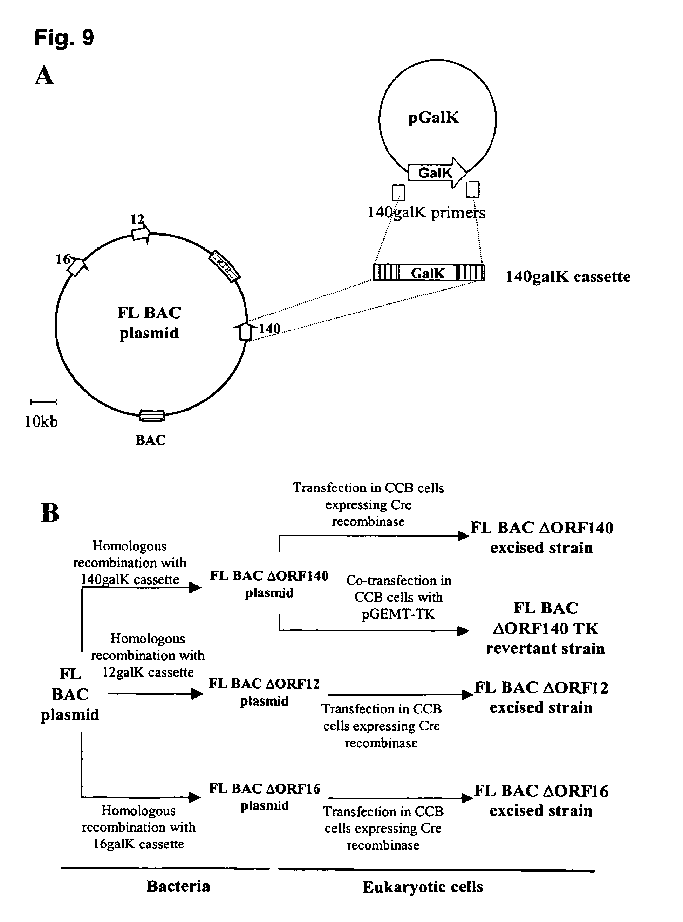

**Figure 9** is a schematic representation of the strategies used to produce infectious KHV FL BAC galK recombinant plasmids and derived viral strains. **(A)** Left part. Schematic representation of the KHV FL BAC plasmid containing a single terminal repeat. The BAC cassette and three ORFs of interest (12, 16 and 140) are indicated. Right part. The 140galK cassette was amplified by PCR using the pGalK vector as template and 140galK primers. In this cassette, the galK ORF is flanked by 50 bp KHV homologous regions. The 12galK and 16galK cassette were produced using the same method. **(B)** Flowchart of stages performed to produce KHV FL BAC galK recombinant plasmids, to demonstrate the possibility of removing the *IoxP*-flanked BAC cassette from the genome of reconstituted virus (FL BAC excised strain) or to produce a wild type revertant strain derived from the FL BAC plasmid (this latter point applies only to ORF140).

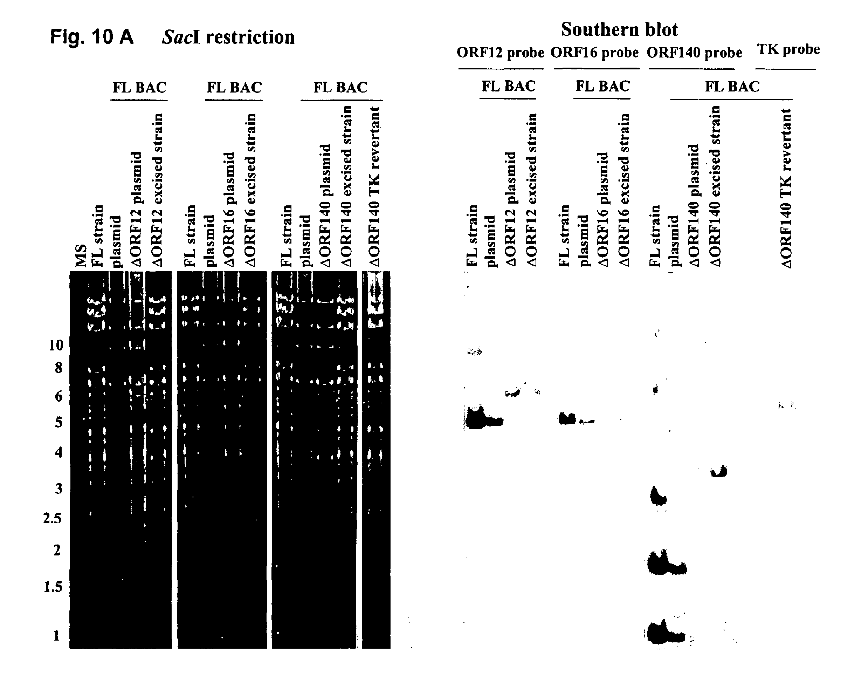

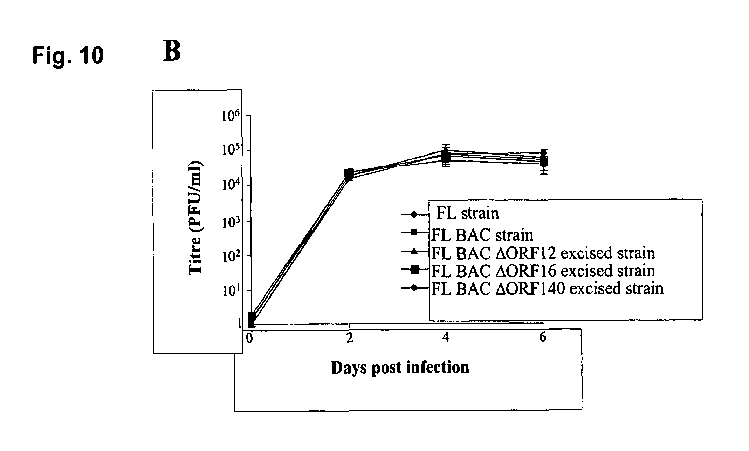

**Figure 10** shows characterization of KHV FL BAC galK recombinant plasmids and derived viral strains. **(A)** Structural analysis of KHV FL BAC galK recombinant plasmids and derived viral strains. The KHV FL BAC plasmid, the derived galK recombinant plasmids and the genome of derived excised or revertant strains were analyzed by *Sac*I restriction (left panel) and further tested by Southern blot (right panel) using probes corresponding to ORF12 (putative tumor necrosis factor (TNF) receptor gene), ORF16 (putative G-protein coupled receptor (GPCR) gene), ORF140 (putative thymidylate kinase gene) or TK ORF. KHV FL strain was used as control. White arrows and open arrows indicate restriction fragments containing the corresponding ORF and galK cassette, respectively. Grey arrow indicates restriction fragment containing TK ORF. Markers sizes (MS) in kb are indicated on the left. **(B)** Replication kinetics of KHV FL BAC galK recombinant strains were compared to those of the parental KHV FL strain as described in materials and methods. The data presented are the means of triplicate measurements.

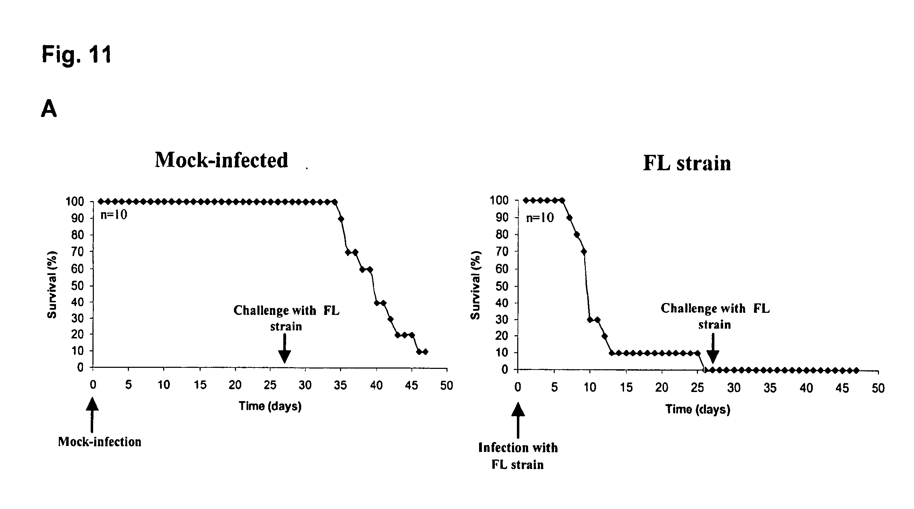

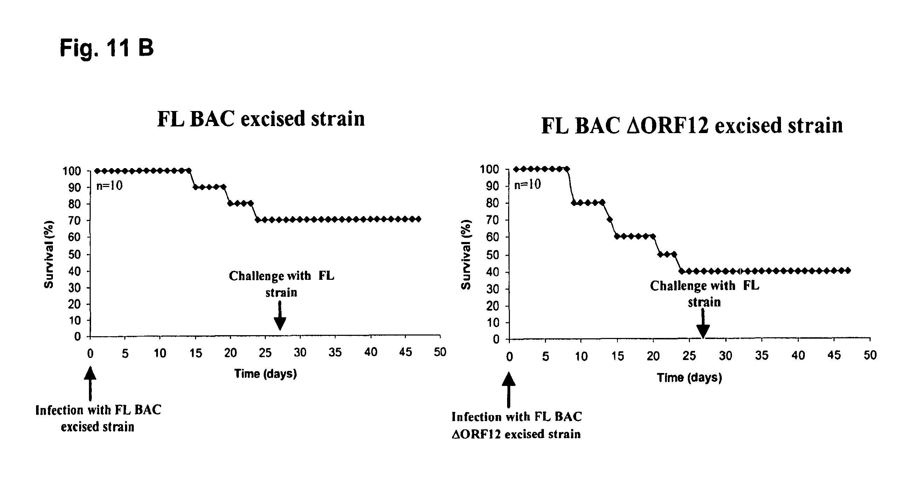

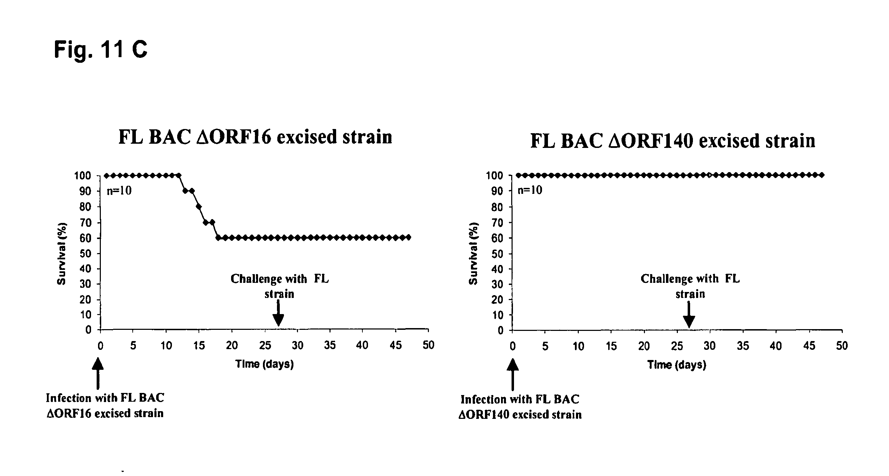

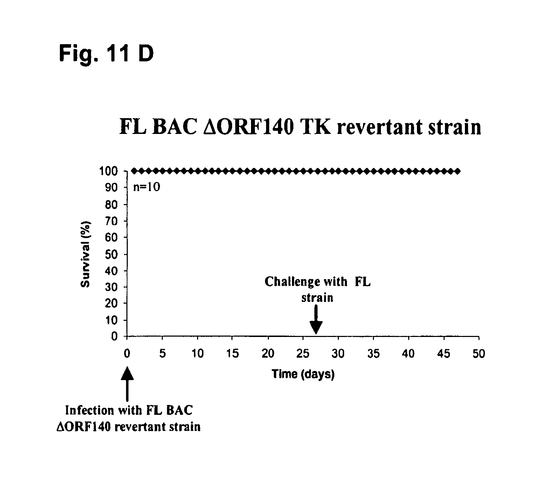

**Figure 11** shows the cumulative incidence of survival rate of carps infected by BAC derived multi-deleted recombinant strains. On day 0, seven groups, each consisting of 10 Koi carps (7 g), were inoculated by IP with mock-infected culture medium **(A)** and culture medium containing 3 x 10² PFU of the FL (TK⁺, TMPK⁺) **(A),** the FL BAC excised (TK⁻, TMPK⁺) **(B),** the FL BAC ΔORF12 excised (TK⁻, TMPK⁺) **(B),** the FL BAC ΔORF16 excised (TK⁻, TMPK⁺) **(C),** the FL BAC ΔORF140 excised (TK⁻, TMPK⁻) **(C)** and the FL BAC ΔORF140 TK revertant strains (TK⁺, TMPK⁻) **(D).** On day 27 post-inoculation, surviving fishes were challenged by IP with the FL strain (3x10² PFU/fish). The percentage of survivors is expressed according to time post infection. The results presented are representative of three independent experiments.

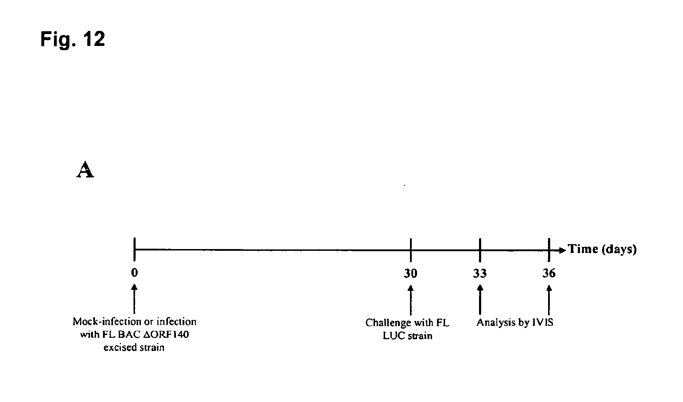

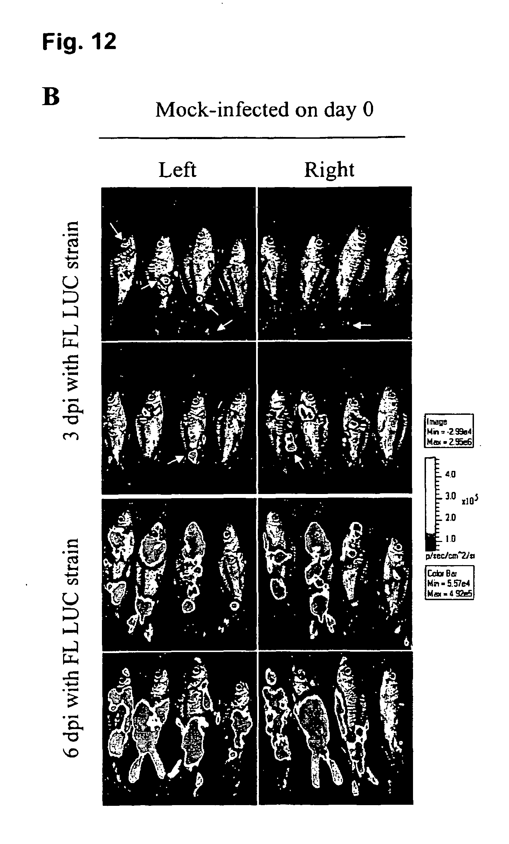

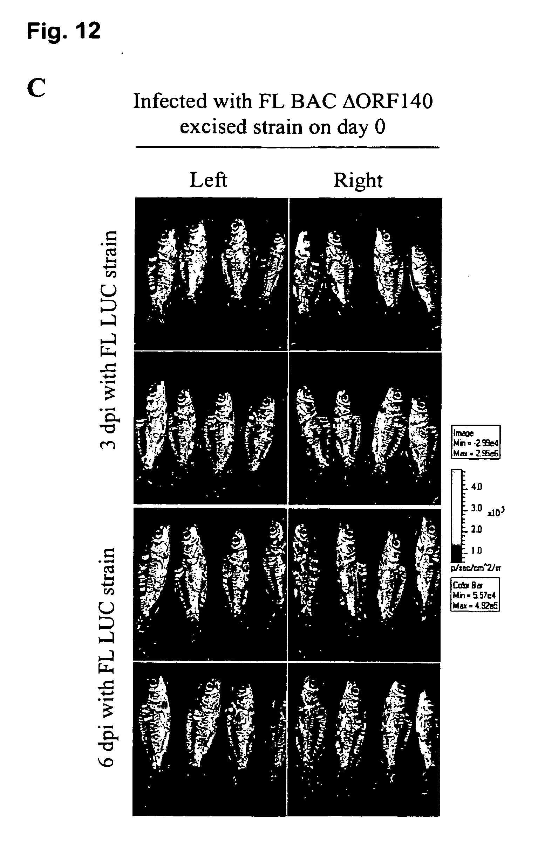

**Figure 12** shows the clinical and virological protection confered by the FL BAC ΔORF140 excised strain using bioluminescence imaging on common carps. **(A)** Schematic representation of the experiment. On day 0, two groups, each consisting of 8 common carps (8 g), were infected by balneation (40 PFU/ml) with mock-infected culture medium or with the FL BAC ΔORF140 excised strain. Thirty days pi, all fishes were challenged by balneation (40 PFU/ml) with the virulent KHV FL LUC strain expressing luciferase under control of human IE promotor. Three and six days post-challenge (33 and 36 dpi) carps were then anesthetized and analysed using *in vivo* imaging system (IVIS). **(B and C)** Bioluminescence imaging. The luciferase activity is depicted with a pseudocolor scale, using red as the highest and violet as the lowest level of photon flux. Representative images are presented with a uniform minimum and maximum threshold value for photon flux. Bioluminescence signals are displayed in pseudocolors and superimposed on the greyscale image.

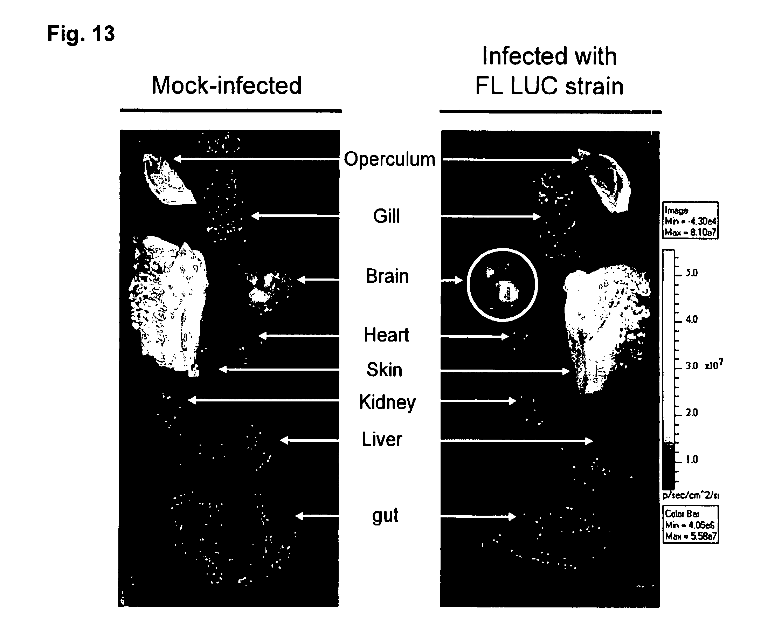

**Figure 13** shows the identification of the brain as the site of KHV latency. Two groups, each consisting of 10 Koi carps (mean weight 27 g), were mock-infected or infected by balneation. For infection, the KHV FL LUC strain was added to water to reach a final concentration of 40 PFU/ml. Fifty days post-infection, carps that survived the acute infection (n=3) were sacrificed, dissected and isolated organs were analysed using *in vivo* imaging system (IVIS). The luciferase activity is depicted with a pseudocolor scale, using red as the highest and violet as the lowest level of photon flux. One representative carp is represented for each group. Representative images are presented with a uniform minimum and maximum threshold value for photon flux. Bioluminescence signals are displayed in pseudocolors and superimposed on the greyscale image. The only organ that emitted detectable signal was the brain (white circle).

### Examples

**Materials and Methods**

**a) Cells and viruses**. Cyprinus carpio brain cells (CCB) (Neukirch et al., Bull. Eur. Ass. Fish. Pathol., 19 (5), 221-224 (1999)) were cultured in minimum essential medium (MEM, Invitrogen) containing 4.5 g/l glucose (D-glucose monohydrate, Merck) and 10 % fetal calf serum (FCS). Cells were cultured at 25°C in a humid atmosphere containing 5 % CO₂. The KHV FL strain was isolated from the kidney of a fish which died from KHV (CER Marloie, Belgium). FL stands for François Lieffrig who isolated the strain. KHV IS and KHV-U strains were kindly provided by M. Kotler (Hebrew University-Hadassah Medical School, Jerusalem, Israel) and R. Hedrick (University of California, School of Veterinary Medicine, USA) respectively (Ronen et al., Vaccine, 21 (32), 4677-4684 (2003); Hedrick et al., Bull. Fish. Res. Agen., S2, 1-7 (2005)).

**b) Growth assay.** Triplicate cultures of CCB cells were infected with KHV FL, KHV IS and KHV-U strains (Multiplicity of infection of 0.5 PFU/cell). After an incubation period of 2 h, cells were washed with PBS and then overlaid with Dulbecco's modified essential medium (DMEM, Invitrogen) containing 4.5 g/l glucose and 10 % FCS. Five days pi, supernatants of infected cultures were harvested and the amount of infectious virus was determined by plaque assay on CCB cells as described previously (Gillet et al., J Gen Virol, 86, 907-917 (2005)).

**c) BAC cloning of KHV.** Firstly, a 1137 bp DNA fragment, corresponding to TK ORF (ORF55) and ORF56 of the KHV genome, was amplified by PCR using KHV FL DNA as template. The following primers were used for the amplification: the forward primer 5'-ATGGCTATGCTGGAACTGGTG-3' and the reverse primer 5'-CTCAACAGGGAAGAGTGGCG-3' corresponding to nucleotides 1-21 of KHV TK ORF and nucleotides 279-297 of ORF56 respectively (Genbank accession no. DQ177346). The amplification product was TA cloned into the pGEMT-easy vector (promega) resulting in pGEMT-TK (Fig. 3A**)**. Three independent clones were sequenced. A BAC cassette was released by Pmel digestion of a pBeloBACModified-EGFPNeo vector (Dewals et al., J Gen Virol, 87, 509-517 (2006)) then ligated into the *Rsr*II site of the pGEMT-TK vector resulting in pGEMT-TKBAC vector (**Fig. 3A**) in which the BAC cassette was flanked by sequences homologous to KHV genome. These homologous regions of 558 bp and 577 bp were used to produce a KHV FL BAC recombinant strain by homologous recombination in eukaryotic cells (**Fig. 3B**). Briefly, freshly seeded CCB cells were infected with KHV at a MOI of 0.5 PFU/cell. After an incubation period of 2 h, cells were transfected with circular pGEMT-TKBAC vector using Lipofectamine Plus (Invitrogen). Four days pi, cell supernatant of infected cells was harvested and inoculated on confluent CCB cell monolayers (10⁶ cells per 9.5 cm²) in the presence of G418 (final concentration 500 µg/ml). This step was repeated three times until a pure BAC-recombinant population was obtained. This viral preparation was inoculated on freshly seeded CCB cells at a MOI of 1 PFU/cell. Circularized form of the viral BAC-recombinant genome was extracted 20 h pi as described previously (Morgan et al., Avian Dis, 34, 345-351. (1990)) and 2 µg DNA was electroporated (2250 V, 132 Ω, 40 µF) into E. *coli* DH10B cells (Invitrogen) as described elsewhere. Electroporated cells were plated on solid LB plate supplemented with chloramphenicol (17 µg/ml). Note that it is crucial at this stage to avoid liquid preculture in order to avoid preferential growing of bacteria containing incomplete KHV BAC plasmid.

**d) Reconstitution of infectious virus from the KHV FL BAC** plasmid. The FL BAC plasmid was transfected (Lipofectamine Plus, Invitrogen) into permissive CCB cells in order to produce the FL BAC recovered strain. To produce a wild type revertant strain derived from the BAC, the FL BAC plasmid was co-transfected in CCB cells together with the pGEMT-TK vector (molecular ratio 1:75). Seven days post-transfection, viral plaques negative for EGFP expression were picked and enriched by three successive rounds of plaque purification. Similarly, to reconstitute virions with excised BAC cassette from the viral genome, the FL BAC plasmid was co-transfected in CCB cells together with the pEFIN3-NLS-Cre vector encoding Cre recombinase fused to a nuclear localization signal (Gillet et al., J Gen Virol, 86, 907-917 (2005)) (molecular ratio: 1:70).

**e) Southern blotting.** Southern blot analysis was performed as described previously (Markine-Goriaynoff et al., J Gen Virol, 85, 355-367 (2004)). Several probes were used. The TK probe was produced by PCR using the TKfw 5'-ATGGCTATGCTGGAACTGGTG-3' and the reverse primer (TKrev) 5'-CTCAACAGGGAAGAGTGGCG-3' corresponding to nucleotides 1-21 of KHV TK ORF and nucleotides 279-297 of ORF56, respectively (Genbank accession no. DQ177346) and KHV FL genome as template. TR probe corresponded to nucleotides 3817-4228 of the LTR and nucleotides 276494-276905 of the RTR of the KHV genome (genbank accession no. DQ177346). The BAC probe was released from the pBeloBACModified-EGFPNeo vector by Pmel digestion. ORF12, ORF16 and ORF140 probes corresponded to the ORF of these locus.

**f) Indirect immunofluorescence staining**. CCB cells were fixed and permeabilized with acetone-ethanol (50:50) for 10 min at -20 °C. Immunofluorescent staining (incubation and washes) was performed in PBS containing 10 % FCS. Samples were incubated at 25 °C for 45 min with a mouse monoclonal antibody (Mab) 8G12 raised against a non identified KHV antigen expressed in the nucleus of infected cells. After three washes, samples were incubated at 25 °C for 30 min with Alexa Fluor 568 goat anti-mouse IgG (H+L) (GAM 568, 2 µg/µl, Molecular Probes) as the secondary conjugate.

**g) Microscopy analysis.** Epifluorescent microscopy analysis was performed with a DMIRBE microscope (Leica) equipped with a DC 300F CCD camera (Leica) as described previously.

**h) Multi-step growth curves.** Triplicate cultures of CCB cells were infected at a MOI of 0.5 PFU/cell. After an incubation period of 2 h, cells were washed with PBS and then overlaid with Dulbecco's modified essential medium (DMEM, Invitrogen) containing 4.5 g/l glucose and 10 % FCS. Supernatant of infected cultures was harvested at successive intervals after infection and the amount of infectious virus was determined by plaque assay on CCB cells as described previously (Gillet et al., J Gen Virol, 86, 907-917 (2005)).

**i) Production of KHV FL BAC recombinant plasmids using galK positive selection in bacteria**. KHV FL BAC recombinant plasmids deleted for either ORF12 (putative tumor necrosis factor (TNF) receptor gene), ORF16 (putative G-protein coupled receptor (GPCR) gene) or ORF140 (putative thymidylate kinase gene) were produced using a galK positive selection in bacteria as previously described (Warming et al., Nucleic Acids Research, 33 (4) (2005)) **(****Fig. 9****)**. The recombination fragment consisted of a galactokinase (galK) gene (1331 bp) flanked by 50 bp sequences corresponding to the beginning and the end of the ORFs to be deleted. These fragments were produced by PCR using the pgalK vector as template. The following primers were used for the amplification: ORF12: forward 5'-ATGAAGCTGCTTGTGATTCTGGGTCTTGGACTCTGCTACC TGGCTGTTCACCTGTTGACAATTAATCATCGGCA-3' and reverse 5'-TCAACTT CTCTTTGGTTTTCTGCACATATTCGTCCCCCCCACGGTTTTCATCAGCACTGT CCTGCTCCTT-3' primer; ORF16: forward 5'-ATGAAACCTCTGGGTCT TTTTGTTTCTGTGCTCGGGCTGCTTGCCCTGTCCCTGTTGACAATTAATCATC GGCA-3' and reverse 5'-TCATAGGACGCCATCGGTTGAGTTCGCTGCGGCT GCGACTCCCAGTCCTCTCAGCACTGTCCTGCTCCTT-3' primer; ORF140: forward 5'-ATGGAACCCGAGCCTCGACAGCAGAACCGCGAGCTCCCTTTCAT GCCTGTTGACAATTAATCATCGGCA-3' and reverse 5'-TCAAGACCATAAAG TGGGAAGGTGTCGTCGAAGCCGTCTCTGGAGTCGTTCAGCACTGTCCTGCT CCTT-3' primer (Genbank accession no. DQ177346). The amplification product was purified (QIAquick Gel Extraction Kit) and stored at -20°C until use. In parallel, 1 µg of KHV FL BAC plasmid was electroporated (2500 V, 100 Ω, 25 µF) into *E*. *coli* SW102 cells. Electroporated cells were grown at 32°C in liquid LB medium with chloramphenicol (17 µg/ml). Next, electrocompetent SW102 cells containing the KHV FL BAC plasmid were electroporated with 2.5 ng of the PCR products described above. Electroporated cells were plated on solid M63 minimal medium supplemented with 20 % galactose and chloramphenicol (17 µg/ml) as described elsewhere to select bacteria in which homologous recombination occurred. Finally, colonies obtained were streaked onto MacConkey indicator plates as described elsewhere to confirm the production of galK positive clones.

**j) Induction of KHV disease in fish**. Specific-pathogen-free Koi and common carps were kept in 60-liter tanks with a water temperature of 24°C. 1/ To test the pathogenicity of KHV field strains *in vivo,* two groups of fish were used each comprising 10 Koi carps and 10 common carps. Koi carps (7 g) were infected by IP injection with 0.1 ml containing 3 x 10² PFU of the KHV IS or the KHV FL strain. Common carps (2 g) were infected by bathing the fish in a 300 ml tank, to which either the KHV IS or the KHV FL strain was added to achieve a final concentration of 30 PFU/ml. After 2 h under these conditions, common carps were transferred into large tanks. 2/ To compare the pathogenicity of KHV FL BAC derived strains, five groups of fish were used each comprising 10 Koi carps (7 g) (with exception of mock-infected group which consisted of 13 carps). Koi carps were infected by IP injection with 0.1 ml containing 3 x 10² PFU of the KHV FL, the KHV FL BAC recovered, the KHV FL BAC excised and the KHV FL BAC revertant strains. The viral inoculums were titrated before inoculation and back-titrated after inoculation to ensure that the doses were equivalent between groups. The control group (mock-infected) was injected with culture medium under the same conditions. Fishes were examined daily for clinical signs of KHV disease and dead fishes were removed. 3/ The pathogenicity of multi-deleted KHV FL BAC ΔORF12, ΔORF16, ΔORF140 excised and FL BAC ΔORF140 TK revertant strains was investigated in Koi carps as described above (see point 2). The animal study was accredited by the local ethics committee of the University of Liège (Belgium).

**k) Production of KHV FL LUC recombinant strain using galK positive and negative selection in bacteria**. A KHV FL LUC recombinant expressing the firefly luciferase was produced as follows. Firstly, a KHV FL BAC plasmid carrying the galK cassette into the intergenic ORF136 and ORF137 region (GenBank accession no. DQ177346) was produced using the KHV FL BAC plasmid and a galK positive selection of bacteria as described above. Secondly, the galK cassette was replaced by a LUC cassette corresponding to the firefly luciferase gene under the control of the human cytomegalovirus immediate-early promoter using a galK negative selection of bacteria (Warming et al., Nucleic Acids Research, 33 (4) (2005)) leading to the KHV FL BAC LUC plasmid. Thirdly, to reconstitute infectious particles encoding the LUC expression cassette and a wild type TK sequence, the KHV FL BAC LUC plasmid was co-transfected into CCB cells with the pGEMT-TK vector previously described, leading to the KHV FL LUC strain. The KHV FL LUC strain was proved to replicate comparably to the parental FL strain *in vitro. In vivo,* the KHV FL LUC and the parental FL strain exhibited comparable virulence when inoculated by bathing to 8 g common carps. Both strains induced mortality rates around 80% (data not shown).

**l) Bioluminescence imaging**. Imaging of firefly luciferase in fish was performed on the Xenogen in vivo imaging system (IVIS) which consists of a cooled charge-coupled device camera (Xenogen). Briefly, 150 mg/kg of D-luciferin (Xenogen) was administrated to fish by IP injection. Fish were then anesthetized with benzocaïne (50 mg/l of water), and imaging began 10 min after administration of D-luciferin. Images were acquired using a 1 min exposure time, a binning factor of 4 and a f/stop of 1. Images were acquired for the left and the right side of each fish. Relative intensities of transmitted light from *in vivo* bioluminescence were represented as a pseudocolor image ranging from violet (least intense) to red (most intense). Images are presented with a uniform minimum and maximum threshold value for photon flux. Corresponding grey-scale photographs and color luciferase images were superimposed with Livinglmage analysis software (Xenogen).

### Example 1: The KHV FL strain has intrinsic properties as a parental strain for production of derived attenuated recombinant vaccines.

Field strains were tested for their growth properties *in vitro* and their virulence *in vivo.* The KHV FL strain was selected at the end of this screening process as parental strain for production of derived attenuated recombinant strains. **Figure 1** illustrates the growth properties of the KHV FL strain as compared to two KHV reference strains (IS and KHV-U strains), the FL strain replicated more efficiently *in vitro.* **Figure 2** shows that *in vivo* the KHV FL strain was less pathogenic than the KHV IS strain. Indeed, when assayed on 7 g Koi carps, the IS and the FL strain led to 90% and 80% mortality, respectively (Fig. 2**)**. Interestingly, when assayed on 2 g common carps, the onset of the disease induced by the FL strain was delayed by two weeks in comparison to the IS strain. In view of the observed delay by two weeks compared to the higher virulent KHV strain and considering the fact that in common carps the onset of the disease caused by the higher virulent KHV strain occurs after about 1 week, the FL strain was selected as parental strain for further experiments (FL stands for François Lieffrig who isolated the strain) [deposition number LMPB 6581CB].

### Example 2: Cloning of the KHV genome in E. coli.

In order to produce KHV recombinant strains, the genome of the KHV FL strain was cloned as a BAC. The approach depicted in **Fig. 3** was used to BAC clone KHV. This approach required as a first step the insertion of the BAC cassette into the KHV genome by homologous recombination in eukaryotic cells. As the genome of KHV was only partially sequenced when the present inventors started their study, the insertion site of the BAC cassette was selected at the end of the TK gene (**Fig.** 3A**)**. Transfection of pGEMT-TKBAC into KHV-infected CCB cells generated the KHV FL BAC strain (**Fig. 3B**). The ability of the latter virus to grow in the presence of G418 allowed its isolation from the parental strain. Moreover, expression of EGFP induced by the KHV FL BAC strain was used to monitor progress of the selection process. After three passages of the virus in the presence of G418, a pure KHV FL BAC population was obtained. The molecular structure of the KHV FL BAC strain was confirmed by a combined SacI restriction endonuclease and Southern blot approach (**Fig. 4**). In the parental FL strain, the TK ORF was contained into a DNA fragment of approximately 5.2 kb. In the FL BAC strain, as a consequence of the BAC cassette insertion into the TK locus, the TK sequence was distributed in two fragments of approximately 5.3 kb and 9.1 kb (**Fig. 4**). Next, we tried to clone circular intermediates of the FL BAC genome into bacteria. Surprisingly, despite the screening of more than 500 independent clones we were unable to select a single clone encoding the FL BAC genome. The BAC plasmids obtained generated heterogeneous restriction profiles with only few bands corresponding to the expected restriction profile (**Fig. 5**). Due to the large size of KHV genome, one could postulate that bacteria encoding incomplete KHV BAC plasmids could have a selective advantage on bacteria encoding a full length KHV BAC clone; and consequently, that liquid culture of transformed bacteria (performed before platting on solid medium) could lead to selection of bacteria with BAC plasmid encoding only part of the KHV genome. To test this hypothesis, E. coli were platted immediately on solid LB medium after electroporation. This approach led to BAC plasmids encoding most of the restriction fragments found in the FL BAC strain genome. However, only 1-2% of the clone exhibited a restriction profile comparable to the FL BAC strain genome. One of these correct clones was characterized by a combined *Sac*I restriction endonuclease and Southern blot approach **(****Fig. 4****).** Due to the circularization of the genome in bacteria, the two bands encompassing the extremities of the left and the right terminal repeats (LTR and RTR) (7.1 kb and 14.1 kb) present in the FL and FL BAC strains are missing in the FL BAC plasmid **(****Fig. 4****).** However, the restriction profile of the FL BAC plasmid did not revealed the expected fused fragment of 21.2 kb **(****Fig. 4****).** This observation suggested that the BAC contains a single copy of the terminal repeat. To test this hypothesis, *Sac*I profiles were analyzed by Southern blotting using the TR probe. This analysis revealed the presence of two bands (7.1 kb and 11.3 kb) in the FL and FL BAC strain profiles and only one single band (11.3 kb) in the FL BAC plasmid **(****Fig. 4****).** These results demonstrate that the FL BAC plasmid contains only a single copy of the terminal repeat. This observation was also confirmed by sequencing of the BAC. All together, the results presented above demonstrate that the entire KHV genome has been cloned as a BAC in *E*. *coli* [Deposition number LMBP 5658].

### Example 3: Stability of the KHV genome in E.coli.

BAC plasmids are usually propagated in bacteria carrying *rec*A mutation that minimizes recombination. However, the large size and the complex structure of KHV genome could lead to a relative instability of the BAC plasmid (Ilouze et al., Microbiol. Mol. Biol. Rev., 70 (1), 147-156, (2006)). To assess the stability of KHV genome as a BAC plasmid, bacteria containing KHV FL BAC plasmid were serially cultured for 20 consecutive days, each day representing approximately 36 generations. After various periods of culture, the BAC plasmids were isolated and characterized by *Sac*I endonuclease digestion **(****Fig. 6****).** No difference was observed among plasmids grown for various periods of time, demonstrating a high stability of the KHV genome in *E*. *coli.*

### Example 4: Reconstitution of infectious virus from the KHV FL BAC plasmid.

The usefulness of a herpesvirus BAC clone requires the ability to reconstitute infectious particles from the BAC plasmid. Consequently, the inventors tested whether infectious particles could be produced by transfection of KHV FL BAC plasmid into CCB cells **(****Fig. 3B****).** Six days post-transfection, viral syncitia expressing EGFP were detected. *Sac*I restriction analysis of the DNA of reconstituted virus (KHV FL BAC recovered strain) revealed restriction profile identical to the pattern observed for KHV FL BAC strain **(****Fig. 4****).** These data demonstrated that infectious KHV FL BAC virions could be reconstituted efficiently by transfection of the KHV FL BAC plasmid into permissive cells. It is important to note that production of reconstituted virions was obtained without any promoting drug treatment. These data demonstrate that the BAC is able to regenerate the entire genome and infectious particles even if it encodes only a single terminal repeat.

To excise the BAC cassette from the genome of reconstituted virions, the FL BAC plasmid was co-transfected into CCB cells together with a Cre recombinase-expressing vector **(****Fig. 3B****).** Deletion of the BAC cassette was monitored by the disappearance of EGFP fluorescence **(****Fig. 7****)** and by a combined restriction endonuclease and Southern blot approach **(****Fig. 4B****).** The cre-I*o*xP-mediated deletion of the BAC cassette left a sequence of 172 bp at the end of TK ORF leading to a *Sac*I restriction fragment slightly larger than the wild type corresponding fragment. The 172 pb consists in one IoxP site (34 bp) and the sequences of the BAC cassette upstream (126 bp) and downstream (12bp) of the IoxP sites. Due to this 172pb insertion of foreign sequence into TK ORF, the FL BAC excised strain expressed a truncated form of TK corresponding to the 185 first amino acids (aa) of the wild type protein (217 residues).

Finally, to generate a revertant strain, the FL BAC plasmid was co-transfected into CCB cells together with the pGEMT-TK vector **(****Fig. 3B****).** The FL BAC Revertant strain was selected on the non expression of EGFP. Restriction analysis revealed a profile identical to the pattern observed for the parental wild type FL strain **(****Fig. 4****).**

### Example 5: Characterization of KHV FL BAC derived strains.

Additional characterization of FL BAC derived strains was performed in cell culture. Firstly, microscopic examination of immunostained viral syncytia did not reveal differences between recombinants **(****Fig. 7A****).** Secondly, in order to investigate the putative effect of the recombination processes on viral growth in vitro, all recombinant strains were compared using a multi-step growth assay **(****Fig. 7B****).** All viruses tested exhibit similar growth curves (P≤0.05) leading to the conclusion that TK disruption does not affect KHV replication in vitro and that KHV genome can support large insertion despite its large size.

### Example 6: BAC derived virus as vaccine.

The BAC cloning strategy of the KHV FL genome was designed to disrupt the Thymidine kinase (TK) locus. To test whether deletion of this ORF would contribute to the attenuation of viruses derived from the KHV FL BAC, the FL BAC recovered and the FL BAC excised strains (which are TK -) were compared to the FL parental and the FL BAC revertant strains (which are TK +) *in vivo.* This experiment was performed in 7 g Koi carps **(****Fig. 8****).** The parental FL strain induced all the clinical signs associated with KHV including apathy, folding of the dorsal fin, increased mucus secretions, suffocation, erratic swimming and lose of equilibrium. The FL strain induced a mortality of 80%. At necropsy, discoloration of gill filaments, herpetic skin lesions and necrotic nephritis were observed on most fishes. In comparison to the FL parental strain, the FL BAC recovered and excised strains exhibited a partially attenuated phenotype characterized by similar clinical signs and lesions but of reduced intensity. Consistent with the attenuation observed, the mortality rate of these strains was reduced to 40%. Importantly, the virulence of the FL BAC revertant strain was similar to that of the parental FL strain. Interestingly, all fishes that survived to the primary infection developed a protective immune response against a further challenge (day 32 pi) with the virulent FL strain. All together these data support the potential of the FL BAC clone as a parental plasmid for the development of multi-attenuated recombinant vaccines.

### Example 7: KHV BAC plasmid as a parental plasmid for the production of KHV BAC recombinant plasmids by mutagenesis in bacteria using galK positive selection.

The results above describe the production of a TK deleted KHV strain (KHV FL BAC excised strain) that induce attenuation of KHV disease when assayed in Koi carps **(****Fig. 8****).** These data support the potential of the FL BAC clone as a parental plasmid for the development of multi-attenuated recombinant vaccines. To demonstrate the feasibility of this concept, three KHV BAC recombinant plasmids, deleted for virulent gene candidates were produced by homologous recombination in bacteria using a galK positive selection: a KHV BAC deleted for ORF12 encoding a putative tumor necrosis factor (TNF) receptor gene. (KHV FL BAC ΔORF12 plasmid); KHV BAC deleted for ORF16 encoding a putative G-protein coupled receptor (GPCR) gene (KHV FL BAC ΔORF16 plasmid) and a KHV BAC deleted for ORF140 encoding a putative thymidylate kinase gene (KHV FL BAC ΔORF140 plasmid) **(****Fig. 9****).** The molecular structure of the produced recombinant plasmids was confirmed by a combined *Sac*I restriction endonuclease and Southern blot approach **(****Fig. 10A****).** In the parental KHV FL strain and KHV FL BAC plasmid, ORF12 and ORF16 were contained into DNA fragments of approximately 4.8 kb whereas ORF140 was contained into two DNA fragments of approximately 1.8 kb and 1 kb. As expected, in KHV FL BAC recombinant plasmid, ORF12, ORF16 and ORF140 DNA fragment encompassing the galK cassette of 1.3 kb was restricted in fragments of approximately 5.7 kb, 5 kb and 3.5 kb, respectively **(****Fig. 10A****).** Note that the bands of 5 kb for ORF16 are slightly visible on the Southern blot. Next, to excise the BAC cassette from the genome of recombinant plasmids, DNAs of KHV FL BAC recombinant plasmids were co-transfected into CCB cells together with a Cre recombinase-expressing plasmid **(****Fig. 9B****).** Deletion of the BAC cassette was monitored by the disappearance of EGFP fluorescence and by a combined restriction endonuclease and Southern blot approach. The cre-loxP-mediated deletion of the BAC cassette left a sequence of 172 bp at the end of TK ORF as described above. Finally, a ΔORF140 TK revertant strain, deleted for ORF140 but containing a wild type transcribed TK sequence was produced **(****Fig. 9B****).** This revertant was selected on the non expression of EGFP. Restriction analysis revealed the presence of the TK ORF as observed for the parental FL strain **(****Fig. 10A****).** Altogether, these results demonstrate that the KHV FL BAC plasmid subject of the present invention can be used to produce recombinant strains using prokaryotic recombination technologies. This result was unexpected since it was hypothesized that the size of the KHV genome and its high content of repeated regions should make this process impossible due to intra-genomic recombination that should occur when turning on the expression of recombinase.

Additional characterization of KHV BAC recombinant strains was performed in cell culture using a multi-step growth assay **(****Fig. 10B****).** All viruses tested exhibit similar growth curves (P≤0.05) leading to the conclusion that disruption of TK coupled to disruption of ORF12, ORF16 or ORF140 does not affect KHV replication *in vitro.*

### Example 8: BAC derived multi-deleted recombinants as vaccine.

The results presented above describe the production of three multi-deleted recombinant strains. To test the potential of these multi-deleted recombinant strains as attenuated vaccines, we compared their pathogenicity and their property to induce a protective immune response against KHV in Koi carps. The results of these experiments are presented in **figure 11****.** The FL BAC ΔORF12 excised strain (deleted for TK and ORF12) and the FL BAC ΔORF16 excised strain (deleted for TK and ORF16) showed a reduced mortality rate compared to the wild-type of 60% and 40%, respectively. Noticeably, the FL BAC ΔORF140 excised strain (deleted for TK and ORF140) induced no mortality and no clinical signs of KHV disease **(****Fig. 11****).** Similarly, the FL BAC ΔORF140 TK revertant strain (deleted for ORF140 but encoding a reverted wild type TK sequence) caused no mortality. Infected fishes exhibited only mild (apathy and folding of the dorsal fin) and temporarily clinical symptoms on day 4 pi. Twenty-seven days post-inoculation, fishes that survived the primary inoculation were challenged with the virulent FL strain. Independently of the strain used for the primary inoculation, all challenged fishes survived without exhibiting any clinical signs of the disease. All together these data support the potential of multi-deleted recombinant strains, particularly the FL BAC ΔORF140 excised strain, as recombinant attenuated live vaccine.

### Example 9: Vaccination of fish with the FL BAC ΔORF140 excised strain confers a clinical and a virological protection against KHV.

The results presented above demonstrate the potential of the FL BAC ΔORF140 excised strain as an attenuated live vaccine. The virus is able to induce a protective immune response against KHV without inducing mortality or even clinical symptoms in vaccinated subjects. Next, we addressed the possibility that vaccination with the FL BAC ΔORF140 excised strain could not only induce an immune protection against KHV disease (clinical protection) but could also confer an immune response preventing further infection (virological protection) when vaccinated subjects are reinoculated by KHV.

To assess this question, common carps (8 g) were first infected by balneation with the FL BAC ΔORF140 excised strain **(****Fig. 12****).** A group of fish infected with culture medium was used as a negative control. Thirty days pi, all fishes were challenged with the virulent KHV LUC strain expressing the firefly luciferase. In order to check the protection conferred by the vaccine strain, all fishes were then analyzed by IVIS 3 and 6 days post-challenge. The results of this experiment are presented in **figure 12****.** Naïve fishes (fishes that were mock-infected on day 0) presented strong bioluminescence signals at day 3 and 6 post-challenge (left panels). On the other hand, fishes vaccinated with the FL BAC ΔORF140 excised strain, did not present any bioluminescent detectable signals at any time post-challenge (right panels). Moreover, these vaccinated fishes, did not present any clinical signs of KHV disease. Altogether, these results support the potential of the FL BAC ΔORF140 excised strain as an attenuated live vaccine conferring clinical and virological protection against KHV.

### Example 10: The FL BAC ΔORF140 excised strain is a vaccine strain that should not be able to reactivate from latency

The site of latency of KHV has been identified as the fish brain **(****Fig. 13****)**. Knowing that brain cells do not divide and consequently do not express cellular TK and TMPK nuclear activities, one could postulate that the TK/TMPK double deleted virus produced (FL BAC ΔORF140 excised strain) should not be able to reactivate from latency. Consequently vaccinated animals should not contribute to the dissemination of the vaccine strain once they are becoming latent carrier.

### References

Aoki, T., Hirono, I., Kurokawa, K., Fukuda, H., Nahary, R., Eldar, A., Davison, A. J., Waltzek, T. B., Bercovier, H. & Hedrick, R. P. (2007). Genome sequences of three Koi herpesvirus isolates representing the expanding distribution of an emerging disease threatening Koi and common carp worldwide. J Virol 81, 5058-65.
Borst, E. M., Hahn, G., Koszinowski, U. H. & Messerle, M. (1999). Cloning of the human cytomegalovirus (HCMV) genome as an infectious bacterial artificial chromosome in Escherichia coli: a new approach for construction of HCMV mutants. J Virol 73, 8320-9.
Dewals, B., Boudry, C., Gillet, L., Markine-Goriaynoff, N., de Leval, L., Haig, D. M. & Vanderplasschen, A. (2006). Cloning of the genome of Alcelaphine herpesvirus 1 as an infectious and pathogenic bacterial artificial chromosome. J Gen Virol 87, 509-17.
Gillet, L., Daix, V., Donofrio, G., Wagner, M., Koszinowski, U. H., China, B., Ackermann, M., Markine-Goriaynoff, N. & Vanderplasschen, A. (2005). Development of bovine herpesvirus 4 as an expression vector using bacterial artificial chromosome cloning. J Gen Virol 86, 907-17.
Hedrick, R. P., Gilad, O., Yun, S.C., MCdowell, T.S., Waltzek, T.B., Kelley, G.O., Adkison, M.A. (2005). Initial isolation and characterization of a herpes-like virus (KHV) from Koi and common carp. Bull. Fish. Res. Agen. Supplement 2, 1-7.
Ilouze, M., Dishon, A. & Kotler, M. (2006). Characterization of a novel virus causing a lethal disease in carp and koi. Microbiol Mol Biol Rev 70, 147-56. Markine-Goriaynoff, N., Gillet, L., Karlsen, O. A., Haarr, L., Minner, F., Pastoret, P. P., Fukuda, M. & Vanderplasschen, A. (2004). The core 2 beta-1,6-N-acetylglucosaminyltransferase-M encoded by bovine herpesvirus 4 is not essential for virus replication despite contributing to post-translational modifications of structural proteins. J Gen Virol 85, 355-67.
Messerle, M., Crnkovic, I., Hammerschmidt, W., Ziegler, H. & Koszinowski, U. H. (1997). Cloning and mutagenesis of a herpesvirus genome as an infectious bacterial artificial chromosome. Proc Natl Acad Sci U S A 94, 14759-63.
Morgan, R. W., Cantello, J. L. & McDermott, C. H. (1990). Transfection of chicken embryo fibroblasts with Marek's disease virus DNA. Avian Dis 34, 345-51.
Neukirch, M., Böttcher, K., Bunnajrakul, S. (1999). Isolation of a virus from Koi with altered gills. Bull. Eur. Ass. Fish. Pathol. 19, 221-224.
Ronen, A., Perelberg, A., Abramowitz, J., Hutoran, M., Tinman, S., Bejerano, I., Steinitz, M. & Kotler, M. (2003). Efficient vaccine against the virus causing a lethal disease in cultured Cyprinus carpio. Vaccine 21, 4677-84.
Wagner, M., Ruzsics, Z. & Koszinowski, U. H. (2002). Herpesvirus genetics has come of age. Trends Microbiol 10, 318-24.
Warming, S., Costantino, N., Court, D. L., Jenkins, N. A. & Copeland, N. G. (2005). Simple and highly efficient BAC recombineering using galK selection. Nucleic Acids Res 33, e36.

## Claims

1. A recombinant Koi Herpesvirus in which the putative thymidilate kinase gene (ORF140) is disrupted, resulting in a KHV which is attenuated and induces a mortality rate of 40% or less in fish.

2. A recombinant Koi Herpesvirus in which the thymidine kinase gene is disrupted, resulting in a KHV which is attenuated and induces a mortality rate of 40% or less in fish.

3. A recombinant Koi Herpesvirus according to claim 1 or 2, in which both the putative thymidilate kinase gene (ORF140) and the thymidine kinase gene are disrupted.

4. A recombinant Koi Herpesvirus according to any of claims 1-3, wherein the Herpesvirus is in a live form.

5. A recombinant Koi Herpesvirus according to any of claims 1-4 which has the capability to reconstitute infectious particles when introduced into permissive eukaryotic cells or fish individuals.

6. A recombinant Koi Herpesvirus according to any of claims 1-5, which results in a mortality rate of 40 % or less, preferably of 20 % or less, more preferred of 10 % or less, further preferred of 1% or less, even further preferred of 0.1% or less and most preferred a mortality rate of 0.0% in fish, preferably in carps, more preferred *Cyprinus carpio carpio* or *Cyprinus carpio Koi* infected with said Herpesvirus.

7. A recombinant Koi Herpesvirus according to any of claims 1-3 or 5-6, which is in inactivated and non-replicative form or in deficient attenuated form.

8. A recombinant Koi Herpesvirus according to any of claims 1-7, comprising a BAC (bacterial artificial chromosome) vector sequence.

9. A recombinant Koi Herpesvirus according to any of claims 1-8, wherein a BAC (bacterial artificial chromosome) vector sequence is inserted into the Koi Herpesvirus genome.

10. A recombinant Koi Herpesvirus according to claim 8 or 9, wherein the BAC vector sequence is excised from the Herpesvirus genome thereby leaving a heterologous sequence at the excision site or former insertion site, respectively, in the Herpesvirus genome.

11. A recombinant Koi Herpesvirus according to any of claims 1-10, further comprising a heterologous sequence or heterologous gene.

12. A recombinant Koi Herpesvirus according to any of claims 1-11, for use as a vector for a heterologous gene.

13. A recombinant Koi Herpesvirus according to claim 12, wherein the heterologous gene is the G glycoprotein of rhabdovirus causing carp spring viraemia.

14. A recombinant Koi Herpesvirus according to any of claims 1-13, further comprising a BAC vector sequence.

15. A cell comprising the recombinant Koi Herpesvirus according to any of claims 1-14.

16. The cell of claim 15 having the deposition number LMBP 5658.

17. A virus produced by the viral genome present in the cell of claim 16.

18. A method for the production of infectious particles of recombinant Koi Herpesvirus (KHV) according to any of claims 1-3, **characterised in that** said method comprises the step of introduction of any of the following recombinant Koi Herpesvirus (KHV) genome into permissive eukaryotic cells: (a) a recombinant Koi Herpesvirus genome (KHV) comprising a bacterial artificial chromosome (BAC) vector sequence inserted in the putative thymidilate kinase gene (ORF140) and/or the thymidine kinase gene ; (b) a recombinant Koi Herpesvirus genome (KHV) wherein the bacterial artificial chromosome (BAC) vector sequence is excised from the construct of (a); and culturing the host cell to produce recombinant Koi Herpesvirus (KHV)

19. Infectious particles generated by the method of claim 18.

20. A vaccine providing immunity against a disease caused by Koi Herpesvirus (KHV), **characterised in that** said vaccine comprises a recombinant Koi Herpesvirus according to any of claims 1-14 or 17, or a DNA sequence comprising the genome of the recombinant Koi Herpesvirus according to any of claims 1-14 or 17.

21. The vaccine according to claim 20 comprising a recombinant Koi Herpesvirus according to any of claims 1-14 or 17.

22. The use of a recombinant Koi Herpesvirus according to any of claims 1-14 or 17 for the manufacture of a medicament/vaccine providing immunity against a disease caused by Koi Herpesvirus.

23. A vaccine providing immunity against a disease caused by rhabdovirus causing carp spring viraemia, which vaccine is comprising a recombinant Koi Herpesvirus according to claim 13, or a DNA sequence comprising the genome of the recombinant Koi Herpesvirus according to claim 13.

24. A recombinant Koi Herpesvirus according to any of claims 1-14 or 17 for use as a vector to transfer a polynucleotide or gene of interest into carp (*Cyprinus carpio*) or cells derived from them or into fish species or cells derived from them other than carp *(Cyprinus carpio).*

## Patentansprüche

1. Rekombinantes Koi-Herpesvirus, in dem das mutmaßliche Thymidilatkinase-Gen (ORF140) unterbrochen ist, was zu einem KHV führt, das abgeschwächt ist und eine Sterblichkeitsrate von 40% oder weniger bei Fischen induziert.

2. Rekombinantes Koi-Herpesvirus, in dem das Thymidinkinase-Gen unterbrochen ist, was zu einem KHV führt, das abgeschwächt ist und eine Sterblichkeitsrate von 40% oder weniger bei Fischen induziert.

3. Rekombinantes Koi-Herpesvirus nach Anspruch 1 oder 2, in dem sowohl das mutmaßliche Thymidilatkinase-Gen (ORF140) als auch das Thymidinkinase-Gen unterbrochen ist.

4. Rekombinantes Koi-Herpesvirus nach einem der Ansprüche 1-3, wobei das Herpesvirus in einer Lebendform vorliegt.

5. Rekombinantes Koi-Herpesvirus nach einem der Ansprüche 1-4, das die Fähigkeit zur Rekonstitution infektiöser Partikel bei Einführung in permissive eukaryontische Zellen oder Fischindividuen aufweist.

6. Rekombinantes Koi-Herpesvirus nach einem der Ansprüche 1-5, das zu einer Sterblichkeitsrate von 40% oder weniger, vorzugsweise von 20% oder weniger, stärker bevorzugt von 10% oder weniger, weiter bevorzugt von 1% oder weniger, noch weiter bevorzugt von 0,1% oder weniger und am stärksten bevorzugt zu einer Sterblichkeitsrate von 0,0% bei Fischen, vorzugsweise bei Karpfen, stärker bevorzugt mit dem Herpesvirus infiziertem Cyprinus carpio carpio oder Cyprinus carpio Koi führt.

7. Rekombinantes Koi-Herpesvirus nach einem der Ansprüche 1-3 oder 5-6, das in inaktivierter und nicht replikativer Form oder in unzulänglicher abgeschwächter Form vorliegt.

8. Rekombinantes Koi-Herpesvirus nach einem der Ansprüche 1-7, umfassend eine BAC(bacterial artificial chromosome)-Vektorsequenz.

9. Rekombinantes Koi-Herpesvirus nach einem der Ansprüche 1-8, wobei eine BAC(bacterial artificial chromosome)-Vektorsequenz in das Koi-Herpesvirus-Genom inseriert wird.

10. Rekombinantes Koi-Herpesvirus nach Anspruch 8 oder 9, wobei die BAC-Vektorsequenz aus dem Herpesvirus-Genom ausgeschnitten wird, wodurch an der Ausschneidstelle bzw. ehemaligen Insertionsstelle im Herpesvirus-Genom eine heterologe Sequenz zurückbleibt.

11. Rekombinantes Koi-Herpesvirus nach einem der Ansprüche 1-10, ferner umfassend eine heterologe Sequenz oder ein heterologes Gen.

12. Rekombinantes Koi-Herpesvirus nach einem der Ansprüche 1-11, zur Verwendung als Vektor für ein heterologes Gen.

13. Rekombinantes Koi-Herpesvirus nach Anspruch 12, wobei es sich bei dem heterologen Gen um das G-Glykoprotein von Rhabdovirus, das die Frühlingsvirämie der Karpfen verursacht, handelt.

14. Rekombinantes Koi-Herpesvirus nach einem der Ansprüche 1-13, ferner umfassend eine BAC-Vektorsequenz.

15. Zellen, umfassend das rekombinante Koi-Herpesvirus nach einem der Ansprüche 1-14.

16. Zelle gemäß Anspruch 15 mit der Hinterlegungsnummer LMBP 5658.

17. Virus, produziert von dem in der Zelle gemäß Anspruch 16 vorliegenden Virusgenom.

18. Verfahren zur Produktion infektiöser Partikel von rekombinantem Koi-Herpesvirus (KHV) nach einem der Ansprüche 1-3, **dadurch gekennzeichnet, dass** das Verfahren den Schritt der Einführung eines der folgenden rekombinanten Koi-Herpesvirus-(KHV-)Genome: (a) eines rekombinanten Koi-Herpesvirus-Genoms (KHV), umfassend eine in dem mutmaßlichen Thymidilatkinase-Gen (ORF 140) und/oder dem Thymidinkinase-Gen inserierte BAC(bacterial artificial chromosome)-Vektorsequenz; (b) eines rekombinanten Koi-Herpesvirus-Genoms (KHV), wobei die BAC(bacterial artificial chromosome)-Vektorsequenz aus dem Konstrukt von (a) ausgeschnitten wird, in permissive eukaryontische Zellen und das Kultivieren der Wirtszelle unter Erhalt von rekombinantem Koi-Herpesvirus (KHV) umfasst.

19. Infektiöse Partikel, erzeugt mit dem Verfahren gemäß Anspruch 18.

20. Impfstoff, der für Immunität gegen eine von Koi-Herpesvirus (KHV) verursachte Erkrankung sorgt, **dadurch gekennzeichnet, dass** der Impfstoff ein rekombinantes Koi-Herpesvirus nach einem der Ansprüche 1-14 oder 17 oder eine das Genom des rekombinanten Koi-Herpesvirus nach einem der Ansprüche 1-14 oder 17 umfassende DNA-Sequenz umfasst.

21. Impfstoff nach Anspruch 20, umfassend ein rekombinantes Koi-Herpesvirus nach einem der Ansprüche 1-14 oder 17.

22. Verwendung eines rekombinanten Koi-Herpesvirus nach einem der Ansprüche 1-14 oder 17 zur Herstellung eines Arzneimittels/Impfstoffs, das/der für Immunität gegen eine von Koi-Herpesvirus verursachte Erkrankung sorgt.

23. Impfstoff, der für Immunität gegen eine von Rhabdovirus, das die Frühlingsvirämie der Karpfen verursacht, verursachte Erkrankung sorgt, wobei der Impfstoff ein rekombinantes Koi-Herpesvirus nach Anspruch 13 oder eine das Genom des rekombinanten Koi-Herpesvirus nach Anspruch 13 umfassende DNA-Sequenz umfasst.

24. Rekombinantes Koi-Herpesvirus nach einem der Ansprüche 1-14 oder 17 zur Verwendung als Vektor zum Transfer eines Polynukleotids oder interessierenden Gens in Karpfen (Cyprinus carpio) oder daraus stammende Zellen oder in von Karpfen (Cyprinus carpio) verschiedene Fischspezies oder daraus stammende Zellen.

## Revendications

1. Virus Herpès de Koï recombinant dans lequel le gène putatif de la thymidylate kinase (ORF140) est interrompu, ce qui conduit à un KHV qui est atténué et induit un taux de mortalité de 40% ou moins chez les poissons.

2. Virus Herpès de Koï recombinant dans lequel le gène de la thymidine kinase est interrompu, ce qui conduit à un KHV qui est atténué et induit un taux de mortalité de 40% ou moins chez les poissons.

3. Virus Herpès de Koï recombinant selon la revendication 1 ou 2, dans lequel à la fois le gène putatif de la thymidylate kinase (ORF140) et le gène de la thymidine kinase sont interrompus.

4. Virus Herpès de Koï recombinant selon l'une quelconque des revendications 1 à 3, dans lequel le virus Herpès est sous forme vivante.

5. Virus Herpès de Koï recombinant selon l'une quelconque des revendications 1 à 4, qui a la capacité de reconstituer des particules infectieuses quand il est introduit dans des cellules eucaryotes permissives ou des individus poissons.

6. Virus Herpès de Koï recombinant selon l'une quelconque des revendications 1 à 5, qui conduit à un taux de mortalité de 40% ou moins, de préférence de 20% ou moins, plus préférablement de 10% ou moins, encore plus préférablement de 1% ou moins, toujours plus préférablement de 0,1% ou moins et le plus préférablement, à un taux de mortalité de 0,0% chez les poissons, de préférence chez les carpes, plus préférablement chez *Cyprinus carpio carpio* ou *Cyprinus carpio Koi,* infectés par ledit virus Herpès.

7. Virus Herpès de Koï recombinant selon l'une quelconque des revendications 1 à 3 ou 5 à 6, qui est sous forme inactivée et non réplicative ou sous forme atténuée défectueuse.

8. Virus Herpès de Koï recombinant selon l'une quelconque des revendications 1 à 7, comprenant une séquence de vecteur BAC (chromosome artificiel bactérien).

9. Virus Herpès de Koï recombinant selon l'une quelconque des revendications 1 à 8, dans lequel une séquence de vecteur BAC (chromosome artificiel bactérien) est insérée dans le génome du virus Herpès de Koï.

10. Virus Herpès de Koï recombinant selon la revendication 8 ou 9, dans lequel la séquence de vecteur BAC est excisée du génome du virus Herpès en laissant de ce fait une séquence hétérologue au niveau du site d'excision ou de l'ancien site d'insertion respectivement, dans le génome du virus Herpès.

11. Virus Herpès de Koï recombinant selon l'une quelconque des revendications 1 à 10, comprenant en outre une séquence hétérologue ou un gène hétérologue.

12. Virus Herpès de Koï recombinant selon l'une quelconque des revendications 1 à 11, destiné à être utilisé en tant que vecteur pour un gène hétérologue.

13. Virus Herpès de Koï recombinant selon la revendication 12, dans lequel le gène hétérologue est la glycoprotéine G d'un rhabdovirus étant à l'origine de la virémie printanière de la carpe.

14. Virus Herpès de Koï recombinant selon l'une quelconque des revendications 1 à 13, comprenant en outre une séquence de vecteur BAC.

15. Cellule comprenant le virus Herpès de Koï recombinant selon l'une quelconque des revendications 1 à 14.

16. Cellule selon la revendication 15, ayant le numéro de dépôt LMBP 5658.

17. Virus produit par le génome viral présent dans la cellule selon la revendication 16.

18. Procédé de production de particules infectieuses de virus Herpès de Koï (KHV) recombinant selon l'une quelconque des revendications 1 à 3, **caractérisé en ce que** ledit procédé comprend les étapes consistant à introduire, dans des cellules eucaryotes permissives, l'un quelconque des génomes de virus Herpès de Koï (KHV) recombinant suivants : (a) un génome de virus Herpès de Koï (KHV) recombinant comprenant une séquence de vecteur de chromosome artificiel bactérien (BAC) insérée dans le gène putatif de la thymidylate kinase (ORF140) et/ou le gène de la thymidine kinase ; (b) un génome de virus Herpès de Koï (KHV) recombinant dans lequel la séquence de vecteur de chromosome artificiel bactérien (BAC) est excisée de la construction du paragraphe (a) ; et cultiver la cellule hôte pour produire un virus Herpès de Koï (KHV) recombinant.

19. Particules infectieuses générées par le procédé selon la revendication 18.

20. Vaccin procurant une immunité contre une maladie causée par le virus Herpès de Koï (KHV), **caractérisé en ce que** ledit vaccin comprend un virus Herpès de Koï recombinant selon l'une quelconque des revendications 1 à 14 ou 17, ou une séquence d'ADN comprenant le génome du virus Herpès de Koï recombinant selon l'une quelconque des revendications 1 à 14 ou 17.

21. Vaccin selon la revendication 20, comprenant un virus Herpès de Koï recombinant selon l'une quelconque des revendications 1 à 14 ou 17.

22. Utilisation d'un virus Herpès de Koï recombinant selon l'une quelconque des revendications 1 à 14 ou 17, pour la fabrication d'un médicament/vaccin procurant une immunité contre une maladie causée par le virus Herpès de Koï.

23. Vaccin procurant une immunité contre une maladie causée par un rhabdovirus étant à l'origine de la virémie printanière de la carpe, lequel vaccin comprend un virus Herpès de Koï recombinant selon la revendication 13, ou une séquence d'ADN comprenant le génome du virus Herpès de Koï recombinant selon la revendication 13.

24. Virus Herpès de Koï recombinant selon l'une quelconque des revendications 1 à 14 ou 17, pour être utilisé en tant que vecteur destiné à transférer un polynucléotide ou un gène d'intérêt dans une carpe (*Cyprinus carpio*) ou des cellules dérivées de celle-ci ou bien dans des espèces de poissons, ou des cellules dérivées de celles-ci, autres que la carpe (*Cyprinus carpio*)*.*
